# EUROPEAN PATENT APPLICATION

(11) **EP 2 735 875 A1**
(43) Date of publication of application: **28.05.2014**
(21) Application number: 12194508.3
(22) Date of filing: 27.11.2012
(51) Int. Cl.: G01N 33/68

(54) **Marker sequences for Neuromyelitis Optica (NMO) and use thereof**

(71) Applicant: Protagen AG, 44227 Dortmund (DE)
(72) Inventor: Göhler, Heike, 44797 Bochum (DE)
(74) Representative: Simandi, Claus

(57) **Abstract**

The present invention relates to new markers for Neuromyelitis Optica (NMO), a method for identifying markers for NMO, the use of the markers identified by the method, diagnostic devices, panels of markers, assays, protein arrays comprising markers for NMO and a method for detecting NMO.

## Description

The present invention relates to new markers for Neuromyelitis Optica (NMO), a method for identifying markers for NMO, the use of markers for NMO identified by the method, diagnostic devices, panels of markers, assays, protein arrays comprising the markers for NMO and a method for identifying NMO.

Protein arrays are gaining increasing industrial importance in analysis and diagnosis as well as in pharmaceutical development. Protein arrays are widely used for example in high throughput screening. The rapid and highly parallel detection of a multiplicity of specifically binding molecules in a single experiment is rendered possible hereby. To produce protein arrays, it is necessary to have the required proteins available.

Another method for screening, e.g. high-throughput screening, is the well established Luminex® or xMAP® Technology. This method is a bead-based multiplex assay for the analysis of hundreds of analytes per well. This technology combines advanced fluidics, optics, and digital signal processing with microsphere technology to deliver multiplexed assay capabilities.

xMAP® Technology uses colour-coded tiny beads ("microspheres", "microsphere particle"). Each bead can be coated with a reagent specific to a particular bioassay, allowing the capture and detection of specific analytes from a sample. Inside an analyzer, e.g. a flow-cytometer like the Luminex® analyzer or Bio-Rad® Bio-Plex® analyzer, a light source excites the internal dyes that identify each bead, and also any reporter dye captured during the assay.

The colour-coded beads are pre-coated with analyte-specific capture antibody for the molecule of interest, than the analyte can be bound to the antibody. Analyte bound to the antibody immobilized to the bead can be quantitatively detected by a fluorescence-labelled detection antibody.

The beads are than read on a dual-laser flow-based detection instrument. One laser classifies the bead and determines the analyte that is being detected. The second laser determines the magnitude of the fluorescence signal, which is in direct proportion to the amount of bound analyte.

Because each bead serves as an individual test, a large number of "different bioassays" can be performed and analyzed simultaneously. And since many readings can be made on each bead set results can be validated.

Different types of beads can be used in this technology, for example MicroPlex® Microspheres. MicroPlex® Microspheres are carboxylated polystyrene micro-particles that have been dyed into spectrally distinct sets (or regions) allowing them to be individually identified by a flow cytometer, e.g. an xMAP® Instrument. MicroPlex® Microspheres are in addition magnetic which allows them to be separated from a solution quickly.

Neuromyelitis optica (NMO) or Devic's disease is an inflammatory demyelinating disease of the CNS with severe optic neuritis and myelitis. In that it is very similar to Multiple Sclerosis (MS), but the relationship has been controversial. The NMO-patients suffer from deterioration of the motor functions and as well from deterioration of visual and sensory function. The identification of NMO is very crucial, as the course of the untreated NMO is worse than the course of MS (Kuhle and Petzold, 2011) and requires therefore early diagnosis and therapy.

Clinical, epidemiological and pathological data have meanwhile demonstrated that MS and NMO are distinct entities. In addition, in 2004 (Lennon et al, 2004) a serum autoantibody has been identified specific for NMO (NMO-IgG), that has been identified as antibody directed against Aquaporin-4 (AQP-4), a high abundant water channel of brain tissue, but as well other tissues.

The revised diagnostic criteria for NMO have been revised in 2006 (Wingerchuk, 2006). These criteria consider optic neuritis and acute myelitis as absolute criteria and two of the following three criteria as supportive for the diagnosis of NMO: negative brain-MRI at disease onset, contiguous signal abnormalities in spinal cord MRI three or more segment in length and positive NMO- (AQP-4) IgG status.

These diagnostic criteria have been established using the final clinical diagnosis, but NMO can be confused with e.g. MS early in the course of the disease, but untreated NMO leads faster to disability than MS. Therefore many groups have tried to develop assays to detect AQP-4 antibodies in patient samples to facilitate early diagnosis and treatment. Today, several assays for the detection of AQP-4 antibodies have been developed, showing a high specificity (95%-100% comparing NMO with MS or healthy control groups), but a much lower sensitivity (30% - 47%, 54%-91%, (Fazio et al (2009), Waters (2008)).

The known marker AQP-4 has several additional disadvantages. For example it is expected, that AQP-4 might be serving as a better marker in women than in men.

In this respect there is further need to increase the sensitivity of NMO-specific assays and existing demand for indication-specific diagnostic means for the detection of NMO, in particular for differentiating between NMO and MS.

The goal of the present invention was to identify additional markers or auto-antibody signatures that can be used to identify NMO-patients with higher sensitivity, either as stand-alone markers or in combination with AQP-4-antibodies.

The present invention provides new markers for NMO and for differentiating between NMO and MS. The identified NMO specific markers of the invention are suitable and can be used for early recognition, detection, diagnosis, prognosis, surveillance of treatment and stratification of patients with NMO and for monitoring of progression or regression of the disease respectively.

The present invention further provides means comprising these new markers for NMO and the use of the new NMO makers, for example the use of one or more of the new markers in panels of markers, diagnostic devices, text kits or protein arrays.

The term "Neuromyelitis Optica" (NMO) and Multiple Sclerosis (MS) are defined e.g., according to Pschyrembel, de Gruyter, 263st edition (2012), Berlin.

According to the invention Neuromyelitis optica (NMO), also known as Devic's disease or Devic's syndrome, is an autoimmune, inflammatory disorder in which a person's own immune system attacks the optic nerves and spinal cord. This produces an inflammation of the optic nerve (optic neuritis) and the spinal cord (myelitis). Although inflammation may also affect the brain, the lesions are different from those observed in the related condition, Multiple Sclerosis. Spinal cord lesions lead to varying degrees of weakness or paralysis in the legs or arms, loss of sensation (including blindness), and/or bladder and bowel dysfunction.

Immunological mechanisms have been implicated as major contributors to the pathological process in NMO. Thus, antibodies may play a critical role in the destructive cascade involved in the pathological process in NMO. Assuming immunological mechanisms involved in NMO and the requirements to specific markers, antibodies are candidates for markers in NMO. Antibodies are highly stable proteins, which are easily accessible e.g. in blood or also saliva and can be easily measured with protein microarrays, ELISA, or other methods. For these reasons they could be seen as a good starting point to find candidates for an early diagnosis, with a high sensitivity and specificity and the ability to monitor disease progression.

A bead-based screening strategy for the discovery of NMO-specific auto-antibodies was developed. Sera samples, clinical and other data of NMO patients, MS-diseased and healthy controls were collected and colour-coded beads displaying different human proteins were used for the detection of NMO-specific auto-antibody signatures in human blood.

The Data for auto-antibody signatures of NMO patients, MS patients and healthy persons (this means persons without MS) was collected and processed by statistical procession analysis thereby leading to the identification of NMO specific marker sequences SEQ ID No. 1 to 263. The new NMO specific markers of the present invention can be used to detect characteristics in the immune profile of NMO patients. They are also suitable for the use to detect differences in the immune profile of different NMO patients and for use in individualized medicine.

It was shown that these NMO specific markers can discriminate NMO patients from reference groups, in particular between persons with NMO and persons with MS and between persons with NMO and persons without MS. The identified markers SEQ ID No. 1 to 263, partial sequences and homologous thereof can therefore be used to separate between patients with NMO, patients with MS, and healthy controls or persons without MS, respectively.

All NMO markers according to the invention were identified by a new statistical approach. This common statistical approach comprises at least two steps: univariate analysis and multivariate analysis. In a preferred embodiment of the invention two different approaches of univariate analysis and one approach of multivariate analysis are applied in order to identify the NMO markers. Finally the results of univariant and multivariant analysis are combined leading to the identification of markers that can differentiate between NMO and MS and markers that can differentiate between NMO and healthy or persons without MS, respectively.

The statistical analysis plan (SAP) underlying the present invention provides a comprehensive and detailed description of strategy and statistical techniques to be used for the analysis of data. The details of the SAP are described in detail below and individual data that illustrate the SAP can be obtained from the examples and tables. A man of skill in the art easily can use and apply this information to identify further suitable markers for NMO.

The purpose of the SAP underlying the present invention was to ensure the credibility of results by pre-specifying the statistical approaches prior to the analysis of data. The SAP follows the principles of the International Conference on Harmonization (ICH) E3, E6, and E9 and the relevant Standard Operating Procedures (SOPs).

In a preferred embodiment, the present invention relates to a method for identifying markers for Neuromelitis Optica (NMO) comprising the steps
a) Expose a marker candidate for NMO to sample(s) of NMO patient(s), measure the bonding of the marker candidate by immunofluorescent assay and determine the median fluorescence intensity (MFI) for the marker candidate;
b) Expose the same marker candidate to control sample(s), measure the bonding of the marker candidate by immunofluorescent assay and determine the median fluorescence intensity (MFI) for the marker candidate;
c) Process MFI data from steps a) and b) by univariate analysis;
d) Process MFI data from steps a) and b) by multivariate analysis;
e) Combine the data obtained by univariate analysis and multivariate analysis and identify thereby markers for NMO.

Univariate analysis is the simplest form of quantitative (statistical) analysis. The analysis is carried out with the description of a single variable and its attributes of the applicable unit of analysis. A basic way of presenting univariate data is to create a frequency distribution of the individual cases, which involves presenting the number of attributes of the variable studied for each case observed in the sample. This can be done for example in a table format, with a bar chart or a similar form of graphical representation.

Multivariate analysis relates to the analysis of multiple variables simultaneously.

In a preferred embodiment of the invention the following statistical approaches are used:

### 1) Univariate analysis based on exploratory statistics and testing:

The easiest approach for univariate analysis is to check for each antigen separately the discriminating power between two groups. Ranking lists of antigens are provided taking into account the p-value of the univariate Mann-Whitney U test, and exploratory summary statistics such as the absolute median fluorescence intensity (MFI) value within groups, the effect size and the fold-change. Univariate TOP candidates for the separation between NMO patients and healthy controls as well as between NMO patients and MS patients can be identified by the univariate analysis.

### 2) Volcano plot

The volcano plot arranges antigens along dimensions of biological relevance and statistical significance. The "edge candidates" in the areas outside the reference lines in the left and right upper corner of the graph show antigens with a high fold-change in either direction and at the same time a low p-value (dots can be marked with numbers). Interesting candidates are than picked up from this type of graph for both comparisons NMO patients vs healthy controls and NMO patients vs MS patients.

### 3) Multivariate analysis - PLS-DA (also called "PPLS-DA")

The aim of the partial least squares discriminant analysis ("PLS-DA" or "PPLS-DA") is to extract relevant linear combinations of the antigens for the discrimination between the predefined groups of NMO patients and healthy controls as well as between NMO patients and MS patients. The PPLS-DA starts with all antigens und results in a TOP-list of antigens. This procedure has been run 200 times in order to identify multivariate candidates. With these candidates, the statistical model was run again and TOP antigens can be picked up according to their importance within the set.

### 4) Combination of analyses

As all of these procedures produce independent ranking and/or TOP lists, the overlap and the union of respective candidates were built for group comparisons and as well for inclusion and exclusion of AQP4.

NMO specific markers were identified by collecting MFI data obtained upon binding of specific markers (e.g. antibodies) to NMO specific substances (e.g. NMO auto-antibodies) in body fluids of NMO patients and processing the obtained MFI data by statistical analysis comprising at least one method of univariate analysis and at least one method of multivariate analysis.

In one embodiment of the method according to the invention procession of MFI data is performed by univariate analysis based on EST (exploratory statistics and testing) and / or volcano plot.

In another embodiment of the method according to the invention univariate analysis of MFI data of a marker candidate comprises one or more parameters selected from p-value, fold change, effect size, Fisher's ration, area under the curve (AUC), median absolute MFI within the group, the univariate Mann-Whitney U test.

In statistical hypothesis testing, the p-value is the probability of obtaining a test statistic at least as extreme as the one that was actually observed, assuming that the null hypothesis is true. When the null hypothesis is rejected, the result is said to be statistically significant.

In statistics, the Mann-Whitney U test (also called the Mann-Whitney-Wilcoxon (MWW) or Wilcoxon rank-sum test) is a non-parametric statistical hypothesis test for assessing whether one of two samples of independent observations tends to have larger values than the other.

In another embodiment of the method according to the invention procession of MFI data by multivariate analysis is performed by partial least squares discriminant analysis (PLS-DA) and/or powered PLS-DA.

Partial least squares regression (PLS regression) is a statistical method that bears some relation to principal components regression. It finds a linear regression model by projecting the predicted variables and the observable variables to a new space.

In another embodiment of the method according to the invention control samples are selected from healthy persons.

Healthy persons in the context of the invention are individuals that have no diagnosis of MS (individuals without MS). Healthy persons are in the healthy state. In a preferred embodiment of the invention healthy persons are individuals that have no diagnosis of an infection or illness at all. In another embodiment of the invention healthy persons might have an infection or illness, however, this other infection or illness must be different from MS.

Within the scope of this invention, "patient" or "person" means any test subject - human or mammal - with the proviso that the test subject is tested for NMO. The term "patient" means a person that has NMO or is tested positive for NMO. The patients are therefore a subgroup of the persons.

In another aspect, the present invention relates to markers for NMO identified by the method according to the invention. In a preferred embodiment the invention relates to markers for MNO identified by a method according to the invention and selected from the group comprising SEQ ID No. 1 to 87 (clone sequences), SEQ ID No. 88 to 175 (RNA sequences), SEQ ID No. 176 to 263 (protein sequences), partial sequences of SEQ ID No. 1 to 263 and homologous of SEQ ID No. 1 to 263, preferably from the group of SEQ ID No. 1 to 16, SEQ ID No. 88 to 104, SEQ ID No. 176 to 192, SEQ ID No. 45 - 63, SEQ ID No. 133 to 161, SEQ ID No. 221 to 239. In a preferred embodiment the invention relates to TOP markers SEQ ID No. 1 to 16 (clone sequence) and / or TOP markers SEQ ID No. 45 to 63 (clone sequence) and the corresponding RNA and/or protein sequences thereof.

In another embodiment the invention relates to markers for discriminating MNO from multiple sclerosis and wherein the markers are identified by a method according to the invention and are selected from the group comprising SEQ ID No. 1 to 44 (clone sequences), SEQ ID No. 88 to 132 (RNA sequences), SEQ ID NO. 176 to 220 (protein sequences), partial sequences and homologous thereof, preferably selected from the group of SEQ ID No. 1 to 16, SEQ ID No. 88 to 104, SEQ ID No. 176 to 192. In a preferred embodiment the invention relates to TOP markers SEQ ID No. 1 to 16, the corresponding RNA sequences SEQ ID No. 88 to 104, and the corresponding protein sequences SEQ ID No. 176 to 192, partial sequences and homologous thereof.

In another embodiment the invention relates to markers for discriminating MNO from healthy state and wherein the markers are identified by a method according to the invention and are selected from the group comprising SEQ ID No. 45 to 87 (clone sequences), SEQ ID No. 133 to 175 (RNA sequence), SEQ ID NO. 221 to 263 (protein sequence), partial sequences and homologous thereof, preferably selected from the group of SEQ ID No. 45 - 63, SEQ ID No. 133 to 161, SEQ ID No. 221 to 239. In a preferred embodiment the invention relates to TOP markers SEQ ID No. 45 to 63, the corresponding RNA sequences SEQ ID No. 133 to 161, and the corresponding protein sequences SEQ ID No. 221 to 239, partial sequences and homologous thereof.

In another embodiment the invention relates to markers for diagnosis of MNO selected form the group comprising SEQ ID No. 1 to 87, SEQ ID No. 88 to 175, SEQ ID No. 176 to 263, partial sequences of SEQ ID No. 1 to 263 and homologous of SEQ ID No. 1 to 263, preferably from the group of SEQ ID No. 1 to 16, SEQ ID No. 88 to 104, SEQ ID No. 176 to 192, SEQ ID No. 45 to 63, SEQ ID No. 133 to 161, SEQ ID No. 221 to 239, partial sequences and homologous thereof.

In another embodiment the invention relates to the use of one or more marker(s) selected from the group comprising sequence SEQ ID No. 1 to 87, SEQ ID No. 88 to 175, SEQ ID No. 176 to 263, partial sequences of SEQ ID No. 1 to 263 and homologous of SEQ ID No. 1 to 263 as diagnostic agent, for use in diagnosis of MNO, for prognosis in NMO, for determination of treatment of NMO, for surveillance of treatment of MNO, for stratification in NMO, for therapy control or prediction of prognosis of NMO covering decisions for the treatment and therapy of the patient, in particular the hospitalization of a patient with NMO, for decision of use, effect and/or dosage of one or more drugs, for use as a therapeutic measure or the monitoring of a course of the disease and the course of therapy, for etiology or classification of NMO optionally together with prognosis.

In a further embodiment of the invention, the markers for NMO according to the invention can likewise be combined, supplemented, fused or expanded likewise with known biomarkers for this indication. In a preferred embodiment of the invention one or more NMO markers of the invention are combined or used together with AQP-4.

Therefore the present invention also relates to the use of the new NMO markers together with other markers, preferably other markers for NMO. The present invention relates for example to the use of combinations of one or more of the new markers SEQ ID No. 1 to 263, partial sequences and/or homologous thereof with AQP-4 as a marker.

In another embodiment the invention relates to a diagnostic agent or test kit comprising one or more marker(s) for NMO selected from the group comprising sequence SEQ ID No. 1 to 87, SEQ ID No. 88 to 175, SEQ ID No. 176 to 263, partial sequences of SEQ ID No. 1 to 263 and homologous of SEQ ID No. 1 to 263, preferably from the group of SEQ ID No. 1 to 16, SEQ ID No. 88 to 104, SEQ ID No. 176 to 192, SEQ ID No. 45 to 63, SEQ ID No. 133 to 161, SEQ ID No. 221 to 239 and optionally further substances and/or additives. In a preferred embodiment AQP-4 is used as additional marker in this connection.

In another embodiment the invention relates to a panel of markers comprising one or more marker(s) for NMO selected from the group comprising sequence SEQ ID No. 1 to 87, SEQ ID No. 88 to 175, SEQ ID No. 176 to 263, partial sequences of SEQ ID No. 1 to 263 and homologous of SEQ ID No. 1 to 263, preferably from the group of SEQ ID No. 1 to 16, SEQ ID No. 88 to 104, SEQ ID No. 176 to 192, SEQ ID No. 45 to 63, SEQ ID No. 133 to 161, SEQ ID No. 221 to 239. In a preferred embodiment AQP-4 is used as additional marker in this connection.

In another embodiment the invention relates to an assay or protein array comprising a panel of marker(s) according to the invention, characterized in that the marker(s) is/are applied to a solid support, in particular a filter, a membrane, a bead or microsphere like for example a magnetic or fluorophore-labelled bead, a silica wafer, glass, metal, ceramics, plastics, a chip, a target for mass spectrometry or a matrix.

In another embodiment the invention relates to the use of a panel of markers according to the invention or an assay or protein array according to the invention for the identification and/or validation of an active agent for the prevention or treatment of NMO wherein the panel or the assay or protein array contains means for detecting a binding success, characterized in that the panel or assay or protein array a.) is brought into contact with at least one substance to be tested and b.) a binding success is detected.

In another aspect the invention relates to a method for detecting MNO comprising the steps
a. providing at least one marker for NMO selected from the group comprising sequence SEQ ID No. 1 to 87, SEQ ID No. 88 to 175, SEQ ID No. 176 to 263, partial sequences of SEQ ID No. 1 to 263 and homologous of SEQ ID No. 1 to 263, preferably from the group of SEQ ID No. 1 to 16, SEQ ID No. 88 to 104, SEQ ID No. 176 to 192, SEQ ID No. 45 to 63, SEQ ID No. 133 to 161, SEQ ID No. 221 to 239,
b. bringing it into contact with body fluid or tissue extract of a person, for example a patient and c. detecting an interaction of the body fluid or tissue extract with the marker(s) from a.).

In another embodiment the invention relates to a target for the treatment and/or therapy of NMO selected from the group comprising sequence SEQ ID No. 1 to 87, SEQ ID No. 88 to 175, SEQ ID No. 176 to 263, partial sequences of SEQ ID No. 1 to 263 and homologous of SEQ ID No. 1 to 263, preferably from the group of SEQ ID No. 1 to 16, SEQ ID No. 88 to 104, SEQ ID No. 176 to 192, SEQ ID No. 45 - 63, SEQ ID No. 133 to 161, SEQ ID No. 221 to 239.

In a further preferred embodiment of the invention, the invention relates to the diagnosis of NMO, wherein at least one marker is selected from the group of sequences SEQ ID No. 1 to 87, SEQ ID No. 88 to 175, SEQ ID No. 176 to 263, partial sequences of SEQ ID No. 1 to 263 and homologous of SEQ ID No. 1 to 263, preferably from the group of SEQ ID No. 1 to 16, SEQ ID No. 88 to 104, SEQ ID No. 176 to 192, SEQ ID No. 45 - 63, SEQ ID No. 133 to 161, SEQ ID No. 221 to 239 and the one or more marker(s) is/are used for detecting one or more auto-antibodies on or from a patient to be examined.

In a further embodiment at least 2 to 5 or 10, preferably 30 to 50 markers or 50 to 100 or more markers are used to determined NMO specific auto-antibodies / NMO specific auto-antibody profiles on or from a patient to be examined, in particular such respectively from the group SEQ ID No. 1 to 87, SEQ ID No. 88 to 175, SEQ ID No. 176 to 263, partial sequences of SEQ ID No. 1 to 263 and homologous of SEQ ID No. 1 to 263, preferably from the group of SEQ ID No. 1 to 16, SEQ ID No. 88 to 104, SEQ ID No. 176 to 192, SEQ ID No. 45 - 63, SEQ ID No. 133 to 161, SEQ ID No. 221 to 239.

In a preferred embodiment, the determination of binding partners (e.g. auto-antibodies) of the NMO specific marker(s) according to the invention is carried out outside the body and the determination is carried out in an ex vivo / in vitro diagnosis. The detection of an interaction of this type can for example be carried out with a probe, in particular by an antibody. The invention therefore likewise relates to the object of providing a diagnostic device or an assay, in particular a protein array, which permits a diagnosis or examination for NMO.

Furthermore, the invention relates to a method for the stratification, in particular risk stratification and/or therapy control and/or of a patient with NMO wherein at least one binding partner to a marker for NMO is determined on a patient to be examined.

Furthermore, the stratification of the patients with NMO in new or established subgroups of NMO or MS is also covered, as well as the expedient selection of patient groups for the clinical development of new therapeutic agents. The term therapy control likewise covers the allocation of patients to responders and non-responders regarding a therapy or the therapy course thereof. The present invention therefore also relates to the use of the markers according to the invention for individualized medicine.

"Diagnosis" for the purposes of this invention means the positive determination of NMO by means of the marker(s) according to the invention as well as the assignment of the patients to NMO. The term diagnosis covers medical diagnostics and examinations in this regard, in particular in-vitro diagnostics and laboratory diagnostics, likewise proteomics and nucleic acid blotting. Further tests can be necessary to be sure and to exclude other diseases. The term diagnosis therefore likewise covers the differential diagnosis of NMO by means of the marker(s) according to the invention and the prognosis of NMO.

"Stratification" or "therapy control" for the purposes of this invention means that the method according to the invention renders possible decisions for the treatment and therapy of the patient, whether it is the hospitalization of the patient, the use, effect and/or dosage of one or more drugs, a therapeutic measure or the monitoring of a course of the disease and the course of therapy or etiology or classification of a disease, e.g., into a new or existing subtype or the differentiation of diseases and the patients thereof.

In a further embodiment of the invention, the term "stratification" covers in particular the risk stratification with the prognosis of an outcome of a negative health event.

The term "marker" for the purposes of this invention means that the protein (polypeptide, peptide) and / or the nucleic acid, e.g. RNA / cDNA / DNA encoding for the polypeptide or protein is significant for NMO. For example, the cDNA or the polypeptide or protein that can be respectively obtained thereof can exhibit an interaction with substances from the body fluid or tissue extract of a patient with NMO (e.g. antigen (epitope) / antibody (paratope) interaction, preferably interaction with an auto-antibody). For the purposes of the invention "wherein at least one marker is selected from SEQ ID No. 1 to 87, SEQ ID No. 88 to 175, SEQ ID No. 176 to 263, partial sequences of SEQ ID No. 1 to 263 and homologous of SEQ ID No. 1 to 263 is determined on a patient to be examined" means that an interaction between the body fluid or tissue extract of a patient and the marker according to the invention is detected. An interaction of this type is, e.g., a bond, in particular a binding substance on at least one marker sequence according to the invention or in the case of a cDNA the hybridization with a suitable substance under selected conditions, in particular stringent conditions (e.g., such as usually defined in J. Sambrook, E. F. Fritsch, T. Maniatis (1989), Molecular cloning: A laboratory manual, 2nd Edition, Cold Spring Harbor Laboratory Press, Cold Spring Harbor, USA or Ausubel, "Current Protocols in Molecular Biology, " Green Publishing Associates and Wiley Interscience, N. Y. (1989)). One example of stringent hybridization conditions is: hybridization in 4 x SSC at 65°C (alternatively in 50% formamide and 4 x SSC at 42°C), followed by several washing steps in 0.1 x SSC at 65°C for a total of approximately one hour. An example of less stringent hybridization conditions is hybridization in 4 x SSC at 37°C, followed by several washing steps in 1 x SSC at room temperature.

According to the invention, substances (binding partners, e.g. auto-antibodies and/or auto-antibody profiles) of this type are constituents of a body fluid, in particular blood, whole blood, blood plasma, blood serum, patient serum, urine, cerebrospinal fluid, synovial fluid or of a tissue extract.

In a further embodiment of the invention, however, the binding partners of the markers according to the invention can be represented in a significantly higher or lower amount or concentration in the body fluid or tissue extract of an NMO patient in comparison to for example the healthy state. The difference in concentration or amount can be determined by the markers according to the invention and indicate NMO. The relative sick/healthy expression rates of the binding partners of the NMO markers according to the invention can hereby determined.

Auto-antibodies that are significant for NMO are either expressed only in case of NMO or the levels of these auto-antibodies vary significantly in case of NMO, e.g. they are more or less expressed in case of NMO in comparison to the levels of the respective autoantibody levels in healthy persons or in comparison to the respective levels in MS. According to the invention the marker can especially be used to determine one or more auto-antibodies or auto-antibody profiles that are specific for NMO, preferably that are specific for early detection of NMO and/or diagnosis of NMO and/or surveillance of the treatment of NMO and/or prognosis of NMO.

Auto-antibody profiles in this respect relate to the amount of one or more auto-antibodies that are specifically expressed, e.g. up- or down-regulated in NMO. The auto-antibody profiles relate therefore in one aspect to the composition (one ore more auto-antibodies) of the profile and in another aspect to the amount or concentration of a particular auto-antibody in NMO.

In one embodiment of the invention the marker binds to / recognizes one or more auto-antibodies that are more or less expressed during development, establishment, therapy and/or progression of NMO. In order to characterize theses specific auto-antibody profiles one or more markers according to the invention can be used / are necessary.

The invention comprises the use of at least one NMO marker. In preferred embodiments of the invention two, three, four, five, six seven, eight, nine or ten or more, e.g. 15 or 20 or more markers are used together or sequentially.

The markers according to the invention are the subject matter of sequence listing and can be clearly identified by the sequences SEQ ID No. 1 - 263 in the sequence listing and from the data in table 1 and table 2.

According to the invention, the markers also cover those modifications of the cDNA sequence and the corresponding amino acid sequence as chemical modification, such as citrullination, acetylation, phosphorylation, glycosylation or poly(A) strand and other modifications known to one skilled in the art.

In a further embodiment of the invention, the marker has a recognition signal that is addressed to the substance to be bound (e.g., antibody, autoantibody, nucleic acid). It is preferred according to the invention for a protein the recognition signal is an epitope and/or a paratope and/or a hapten and for a cDNA is a hybridization or binding region. In a preferred embodiment of the invention the marker recognizes (e.g. hybridizes, binds) to an autoantibody which is significant for NMO.

Homologous according to the invention are homologous protein / peptide or nucleic acid sequences, in particular homologous of SEQ 1-263 that display an identity of at least 70 % or 80 %, preferred 90 % or 95 %, most preferred 96 % or 98 % or more, e.g. 98 % or 99 % homology with the respective protein, peptide or nucleic acid sequences or the respective partial sequence.

Partial sequences according to the invention are parts of the respective protein / peptide sequences, in particular those encoded by SEQ 176-263 and the nucleic acids encoding theses partial proteins, peptides. Partial sequence miss one or more amino acids or nucleotides respectively in comparison to the respective complete sequences. The/these missing part(s) could be located at the beginning, the end or within the sequence. Enclosed are also sequences that contain additional sequence parts at the beginning, the end or within the sequence in comparison to the respective complete sequences.

In a further embodiment of the invention, partial sequences of the markers according to the invention are likewise covered. In particular those partial sequences that have an identity of 95%, 90%, in particular 80% or 70% with the markers according to the invention.

Another object of the invention relates to an arrangement of markers (panel) containing at least one marker selected from the group of sequences SEQ ID No. 1 to 87, SEQ ID No. 88 to 175, SEQ ID No. 176 to 263, partial sequences of SEQ ID No. 1 to 263 and homologous of SEQ ID No. 1 to 263, preferably from the group of SEQ ID No. 1 to 16, SEQ ID No. 88 to 104, SEQ ID No. 176 to 192, SEQ ID No. 45 - 63, SEQ ID No. 133 to 161, SEQ ID No. 221 to 239, partial sequences or homologous thereof. Preferably, the arrangement contains at least 2 to 5 or 10, preferably 30 to 50 markers or 50 to 100 or more markers.

In a further embodiment, the respective marker can be represented in different quantities in one more regions in a panel e.g. on a solid support. This permits a variation of the sensitivity. The regions can have respectively a totality of markers, i.e. a sufficient number of different markers, in particular 2 to 5 or 10 or more and optionally additional nucleic acids and/or proteins, preferably AQP-4. However, at least 96 to 25,000 (numerical) or more from different or identical markers and further nucleic acids and/or proteins, in particular AQP-4 is preferred. Furthermore preferred are more than 2,500, in particular preferred 10,000 or more different or identical markers and optionally further nucleic acids and/or proteins, in particular AQP-4.

Within the scope of this invention, "arrangement" is synonymous with "panel" and "array" and if this "array" is used to identify substances or binding partners for the marker(s), this is to be understood to be an "assay" or diagnostic device.

In a preferred embodiment, the arrangement is designed such that the marker(s) represented on the arrangement are present in the form of a grid on a solid support. Furthermore, those arrangements are preferred that permit a high-density arrangement of protein binders and the markers are spotted. Such high-density spotted arrangements are disclosed, for example, in WO 99/57311 and WO 99/57312 and can be used advantageously in a robot-supported automated high-throughput method.

As used herein, the word "array" or "panel" shall be taken to mean any ordered arrangement of a plurality of specified integers, including both linear and non-liner arrangements of a plurality of proteins and/or nucleic acids. In the present context, the word "array" or "panel" includes any elements derived e.g. from a complex mixture of proteins/ nucleic acids resolved by 1-dimensional or 2-dimensional gel electrophoresis or chromatography, or peptide or protein expression libraries and the ordered arrangement of the proteins or nucleic acids on a grid, such as in microtitre wells or on a membrane support or silicon chip or on a grid comprising a plurality of polymeric pins and / or on beads, e.g. magnetic beads.

The solid support or matrix is typically glass or a polymer, the most commonly used polymers being cellulose, polyacrylamide, nylon, polystyrene, polyvinyl chloride or polypropylene. The solid supports may be in the form of tubes, beads, discs, silicon chips, microplates, polyvinylidene difluoride (PVDF) membrane, nitrocellulose membrane, nylon membrane, other porous membrane, non-porous membrane (eg. plastic, polymer, perspex, silicon, amongst others), a plurality of polymeric pins, or a plurality of microtitre wells, or any other surface suitable for immobilising proteins and/or nucleic acids and/or conducting an assay. The binding processes are well-known in the art and generally consist of cross-linking, covalently binding or physically adsorbing the protein or nucleic acid molecule to the solid support.

In all of the embodiments, the term "solid support" covers embodiments such as a filter, a membrane, a bead, preferably a magnetic or fluorophore-labeled bead, a silica wafer, glass, metal, ceramics, plastics, a chip, a target for mass spectrometry or a matrix. However, a filter is preferred according to the invention.

As a filter, furthermore PVDF, nitrocellulose or nylon is preferred (e.g., Immobilon P Millipore, Protran Whatman, Hybond N+ Amersham).

In another preferred embodiment of the arrangement according to the invention, the arrangement corresponds to a grid with the dimensions of a microtiter plate (8 - 12 wells strips, 96 wells, 384 wells or more), a silica wafer, a chip, a target for mass spectrometry, or a matrix.

Within the scope of this invention, however, the term "assay" or diagnostic device likewise comprises those embodiments of a device, such as ELISA, bead-based assay, line assay, Western Blot, immunochromatographic methods (e.g., lateral flow immunoassays) or similar immunological single or multiplex detection measures. A protein array in accordance with the invention is a systematic arrangement of proteins on a solid support or a matrix.

The markers of the arrangement are preferably fixed on a solid support, for example spotted or immobilized or printed on, i.e. applied in a reproducible manner. One or more markers can be present multiple times in the totality of all markers and present in different quantities based on one spot. Furthermore, the markers can be standardized on the solid support.

The invention therefore further relates to an assay or a protein array comprising an arrangement containing markers according to the invention.

In a further embodiment, the markers are represented as clones. Clones of this type can be obtained, for example, by means of a cDNA expression library according to the invention (Büssow et al. 1998 (supra)). In a preferred embodiment, such expression libraries containing clones are obtained using expression vectors from a cDNA expression library comprising the cDNA marker sequences. These expression vectors preferably contain inducible promoters. The induction of the expression can be carried out, e.g., by means of an inductor, such as IPTG. Suitable expression vectors are described in Terpe et al. (Terpe T Appl Microbiol Biotechnol. 2003 Jan; 60(5) : 523-33).

One skilled in the art is familiar with expression libraries, they can be produced according to standard works, such as Sambrook et al, "Molecular Cloning, A laboratory handbook, 2nd edition (1989), CSH press, Cold Spring Harbor, New York. Expression libraries are also preferred which are tissue-specific (e.g., human tissue, in particular human organs). Furthermore included according to the invention are expression libraries that can be obtained by exon-trapping. A synonym for expression library is expression bank.

Also preferred are protein arrays (protein microarrays, protein biochips) or corresponding expression libraries that do not exhibit any redundancy (so-called: Uniclone® library) and that may be produced, for example, according to the teachings of WO 99/57311 and WO 99/57312. These preferred Uniclone libraries have a high portion of nondefective fully expressed proteins of a cDNA expression library.

Within the context of this invention, the clones can also be, but not limited to, transformed bacteria, recombinant phages or transformed cells from mammals, insects, fungi, yeasts or plants.

The clones are fixed, spotted or immobilized on a solid support. The invention therefore relates to an arrangement wherein the markers are present as clones.

Additionally, the markers can be present in the respective form of a fusion protein, which contains, for example, at least one affinity epitope or tag. The tag may be one such as contains c-myc, his tag, arg tag, FLAG, alkaline phosphatase, VS tag, T7 tag or strep tag, HAT tag, NusA, S tag, SBP tag, thioredoxin, DsbA, a fusion protein, preferably a cellulose-binding domain, green fluorescent protein, maltose-binding protein, calmodulin-binding protein, glutathione S-transferase or lacZ.

In a further embodiment, the invention relates to an assay or a protein array for identifying and characterizing a substance for NMO, characterized in that an arrangement or assay according to the invention is a.) brought into contact with at least one substance to be tested and b.) a binding success is detected. The substance to be tested can be any native or non-native biomolecule, a synthetic chemical molecule, a mixture or a substance library. After the substance to be tested contacts a marker, the binding success is evaluated, which, for example, is carried out using commercially available image analyzing software (GenePix Pro (Axon Laboratories), Aida (Ray test), ScanArray (Packard Bioscience).

The visualization of protein-protein interactions according to the invention (e.g., protein on marker, as antigen/antibody, e.g. autoantibody) or corresponding "means for detecting the binding success" can be performed, for example, using fluorescence labelling, biotinylation, radioisotope labelling or colloid gold or latex particle labelling in the usual way. A detection of bound antibodies is carried out with the aid of secondary antibodies, which are labelled with commercially available reporter molecules (e.g., Cy, Alexa, Dyomics, FITC or similar fluorescent dyes, colloidal gold or latex particles), or with reporter enzymes, such as alkaline phosphatase, horseradish peroxidase, etc., and the corresponding colorimetric, fluorescent or chemiluminescent substrates. Readout is conducted, e.g., using a microarray laser scanner, a CCD camera or visually.

In a further embodiment, the invention relates to a drug/active substance or prodrug developed for NMO and obtainable through the use of the assay or protein array according to the invention.

In a further embodiment, the invention likewise relates to the use of the marker according to the invention, preferably in the form of an arrangement, as an affinity material for carrying out an apheresis or in the broadest sense a blood lavage, wherein substances from body fluids of a patient with NMO, such as blood or plasma, bind to the markers according to the invention and consequently can be selectively withdrawn from the body fluid.

The invention is further described in the following examples and tables, however, without restricting the invention to these examples and tables. All of them are generated according to the SAP of the invention. All tables and data listings are presented in Landscape Orientation.

### Examples

### Example 1: Measurement principle

In this study, the bead-based Luminex xMAP® Technology is used for the detection of auto-antibodies in serum samples derived from endometriosis patients.

In a bead-based assay, a set of different fluorescent colour-coded magnetic polystyrene microspheres (MagPlex^{®}) is used, allowing the simultaneous analysis of up to 500 analytes in a single approach by the Luminex FlexMap3D device. Each colour-coded bead is covalently linked to a specific antigen. During serum sample incubation, autoantibodies present in the serum sample bind to the coupled antigens on the beads and can be quantitatively detected by a fluorescence-labelled (e.g. phycoerythrin) detection antibody.

The Luminex FlexMap3D device is based on the principle of flow cytometry using a dual-laser system to identify the specific bead colour with a 635 nm red laser and the signal strength of reporter molecules with a 532 nm green laser.

### 1.1. Coupling Procedure

Histidine-tagged antigens are purified under denaturing conditions and cross-linked to magnetic microspheres (MagPlex^{®}) using standard bead coupling procedure (WI 3-17-1.03 Ver01). The coupling reaction is performed in a semi-automated fashion with a Freedom Evo^{®} 150 liquid handling roboter (Tecan). The carboxylated beads are activated with EDC and Sulfo-NHS and up to 12.5 µg of protein is subjected to the activated beads forming covalent peptide bonds between the carboxyl groups of the bead and primary amines of the protein.

Proteins, which will be coupled, have to fulfil following conditions to enable successful bead coupling and quality control.

### Requirements of protein conditions

| | |
|---|---|
| Buffer conditions: | amine-free buffer (w/o Tris, lysine, glycine, ethanolamine); pH < 8.0 |
| Protein amount: | 100 µg |
| Protein concentration: | > 0.25 µg/µl |
| Protein conditions: | HIS-tagged proteins |
| Protein detection (QC) : | Penta-HIS antibody |

Coupled beads of 400 different colour-codes are combined to a bead mix. The bead mixes are stored at 4°C in the dark. The coupling efficiency is controlled according to the working instruction (WI 3-17-2.02 Ver02) using an anti-penta-His antibody (Qiagen) and a secondary PE-conjugated anti-mouse antibody (Dianova).

Internal control beads are used to monitor the assay performance of each well. Hence, beads coupled with human IgG (Sigma) and mouse IgG (Sigma) are added to each bead mix controlling the accuracy of goat-anti-human-PE or goat-anti-mouse-PE detection antibodies, respectively.

### 1.2. Antigen-Autoantibody Assay

The antigen-autoantibody assay is performed according to the working instructions (WI 3-17-3.03 Ver01, WI 3-17-3.04 Ver02) in a semi-automated fashion using the liquid handling workstations MICROLAB^{®}STARlet (Hamilton) and Freedom Evo^{®} 150 (Tecan).

Serum samples are thawed and re-arrayed in the appropriate format for the bead-based assay using the MICROLAB^{®} STARlet followed by a 1:100 dilution in assay buffer using the Freedom Evo^{®} 150.

The bead mix is analyzed with all serum samples. After distributing the bead mixes in the microtiter plates the 1:100 diluted serum samples are applied for 22 hours at 4°C. Unbound human auto-antibodies of the serum samples are removed by washing. Bound human auto-antibodies are quantitatively labelled by a PE-labelled goat-anti-human IgG antibody (Dianova) followed by washing cycles of the beads. The median fluorescence intensities (MFI) of the detection antibody is analyzed for each bead using the FlexMAP 3D instrument.

For the calculation of intra- and inter-plate CVs three replicates of selected serum samples are measured on each assay plate.

### Example 2: Study Design

### 2.1. Analysis Groups

There are three different analysis groups within the study. The following groups will be considered (n = number of different samples, each sample belongs to a different patient/person):
NMO (n=12)
MS (n=18)
healthy controls (n=12)

### 2.2. Randomization and Blinding

This is an open study, so no blinding on a patient level is possible. Nevertheless, allocation of serum samples on plates followed a block randomization scheme in which matched pairs (patient and control, if applicable) were randomly arranged.

### 2.3. Measurement Quality

The entire processes including bead coupling, coupling control and the antigen-autoantibody assay are performed according to Protagen's working instructions.

Coefficients of variation (CV) will be determined and will be shown for inter- and intra-plate variation in form of histograms and boxplots. Besides, the bead count distribution will be presented analogously.

### 2.4. Analysis Population

All samples available according to the setting described above will be included, no further definition of analysis populations is necessary for this exploratory approach.

### Example 3: Collection and Procession of data

### 3.1. Data Base

Laboratory raw data are available in CSV-format showing relevant MFI values by sample number. Additionally, demographic data are available such as age and gender. All data will be transferred to the R environment for statistical evaluation. Analysis data sets will be generated in the context of data management.

In total, the data base consists of 42 samples: 12 NMO samples, 12 healthy controls, and 18 MS samples coming from one screen.

No course over time will be monitored, i.e. one observation per patient or healthy control is available.

In total, 384 antigens were documented, thereof 247 will be considered for the analysis.

### 3.2. Analysis Variables

The MFI of the detection antibody is the target variable for all analyses. Values are considered for 247 antigens in total. Demographic data will only be used to check homogeneity within the analysis population. No further variables will be considered.

The aim of this study is to answer the question whether the immune profile is able to separate between patients with NMO, patients with MS, and healthy controls. Special interest is on AQP-4 as a marker and potentially accompanying additional or alternative markers.

Therefore, the following objectives will be addressed:
The immune profile for different indications will be investigated and compared. Possible discrimination between the following groups will be analyzed:
   NMO vs healthy controls and NMO vs MS

### 3.3. Data Pre-processing

### 3.3.1. Replicates

If replicates are present, the first measurement values will be used for statistical analyses.

### 3.3.2. Transformation

For MFI values are log₂ transformed before normalization. As long as the statistical method requires log-transformed values, the transformed values are maintained. All other analyses are based on the back-transformed MFI values.

### 3.3.3. Handling of Missing Values

Total amount of employed beads is optimized, targeting at 100 beads counted for each measured antigen. Observations from previous studies show that MFI values are unreliable for a specific antigen if less than 10 beads are counted. These cases are rare, and the corresponding MFI value is set to missing. The numbers of missing values will be reported for each antigen and sample. Samples or antigens are discarded from further analysis if
1) there are more than 20% missing values for a sample,
2) there are more than 20% missing values for an antigen.

Samples or antigens excluded from further analysis will be reported.

Missing values for an antigen will be replaced after normalization and back-transformation by median imputation, i.e. by the median MFI value measured in all samples for this antigen.

### 3.3.4. Normalization

Normalization is applied after log₂ transformation. On each Luminex plate, three reference sera are measured serving as quality control and normalization reference for plate-specific measurement differences. To this end, the median of each antigen is calculated from all reference samples on a plate, yielding the median reference for this individual plate. An overall median reference based on all measured plates is calculated analogously. Quantile normalization is used to normalize all measured samples on the individual plate by BBA set.

### 3.3.4. Statistical Analysis

The complete statistical analysis will be carried out for two group comparisons:
● NMO vs. healthy controls
● NMO vs. MS

Multivariate methods will only be applied as long as the number of samples is sufficient.

### Example 4: Statistical Analysis

### 4.1. Univariate Analysis

Summary statistics will be determined for all MFI results for all antigens separately for all three groups: NMO, MS and healthy controls. (Note: not log-transformed values but original values to be used)

The median will be determined as a representative parameter for location.

Group comparison will be performed between results derived from NMO patients in comparison to healthy controls, and additionally in comparison to MS patients. The following system of hypotheses will be investigated for the log₂ transformed MFI values for each antigen j, j = 1, ..., J:
H₀ⱼ: The medians of log₂ transformed MFI values are identical in the two groups.
H₁ⱼ: The medians of log₂ transformed MFI values differ between the two groups.

Besides, the fold change (=ratio) between the two groups will be determined.

The effect size will be calculated as the ratio of the absolute value of mean difference between the two groups and the respective standard deviation.

A receiver operating characteristic (ROC) curve will be constructed. Sensitivity, specificity and the area under the curve (AUC) will be estimated together with the respective bootstrapped 95% confidence intervals.

The TOP candidates will be identified for further investigation taking into account the following characteristics as decision criteria:
- P-value (TOP 30 rank)
- Fold change (at least 2-fold)
- Effect size (TOP 30 rank)
- Fisher's ratio (TOP 30 rank)
- AUC resulting from ROC analysis (at least 0.75)
- Median absolute MFI value in at least one treatment group >500
- Median absolute MFI value in at least one treatment group >1000

A scoring system will be implemented: The criteria mentioned above will be treated as binary variables, so that one scoring point will be given if the respective criterion is fulfilled. The score will present the sum of all scoring points.

TOP candidates will be ranked according to this scoring system. In case of ties the second variable for ranking is the p-value, so that a clear cut-off for the univariate TOP 30 candidates within the list is possible.

The ranking list will be provided for the comparison between NMO and healthy controls and additionally for the comparison between NMO and MS.

### 4.2. Graphical Display

The volcano plot will visualize a part the univariate results for all antigens at a glance. A volcano plot arranges antigens along dimensions of biological relevance and statistical significance. The horizontal dimension is the fold change between the two groups on a log₂ scale, so that up and down regulation appear symmetric, and the vertical axis represents the p-value for a Mann-Whitney U test of differences between samples on a negative log₁₀ scale, so that smaller p-values appear higher up. The horizontal axis indicates biological relevance of the difference, the vertical axis indicates the statistical significance. Judgment about promising candidates is possible by trading off both these criteria by eye. Reference lines are implemented at -1 and 1 (reflecting a 2-fold change in either direction) on the horizontal axis and at 1.3 (reflecting a p-value of 0.05) on the vertical axis.

The "edge candidates" coming from the areas outside the reference lines (left and right upper corner) will be listed with gene-ID, gene name and log₂ (ratio) and -log₁₀ (p-value)

Graphs and listings will be provided for the comparison between NMO patients and healthy controls, and the same visualizations will be given for NMO patients in comparison to MS patients.

### 4.3. Multivariate Analysis

Partial least squares discriminant analysis (PLS-DA) is partial least squares regression adapted to classification tasks. The aim is to extract relevant components (linear combination of the variables) for the discrimination between the predefined groups. This technique is especially suited to deal with a much larger number of predictors than observations and with multicollinearity, two of the main problems encountered when analyzing expression data.

Powered partial least squares discriminant analysis is a specialized version of the method PLS-DA. One aspect different from PLS-DA is, that a so called power parameter is fitted in order to maximize the correlation between the latent components and the response matrix (dummy coded group memberships). For the final classification a linear discriminant analysis is applied with the latent components as predictors.

The PLS-DA will start with all antigens und result in a TOP-list of 30 antigens. Cross validation will be implemented with a number of 200 runs.

An evaluation over these 200 runs will summarize the ranking based on the frequency of TOP 30 ranks for each antigen and the median value for the rank. An antigen is qualified for the multivariate panel if the frequency of TOP 30 ranks is at least 100, or the frequency is at least 100 and the median rank is not higher than 16.

For this panel a PLS-DA (also "PPLS-DA") will be applied with focus on the first component and the results will be shown in form of a score plot, an importance plot, and a ranking list based on the loading weights.

As the number of samples available is very small, this analysis will be carried out only as supportive. On the one hand all antigens will be used within the PPLS-DA, on the other hand AQP-4 will be left out to investigate a possible difference. Graphical display of results will be provided.

The two groups for comparison will be NMO and healthy controls. An analysis for NMO patients in comparison to MS patients will be carried out analogously.

### 4.4. Combination of Results

In order to get an overview of the candidate lists based on different statistical analyses the results will be pooled. The overlap of panels from univariate (Scoring and "Edge candidates" resulting from Volcano plot) and multivariate analysis will be presented for the two comparisons NMO patients versus healthy controls and NMO patients versus MS patients. Analogously, the respective union of panels will be presented.

### 4.5. Software

In-house developed software is used to perform Luminex raw data file parsing to produce an easy readable CSV file that is suitable as input for further statistical analysis software. All statistical analyses are performed within the R project for statistical computing, http://www.r-project.org/ version R-2.14.0 (2011-10-31).

**Table 1: Markers for NMO identified by statistical analysis of MFI data from NMO vs MS. The sequence of the markers can be obtained from the enclosed sequence listening.**

| SEQ ID No. | Marker Classification | GeneID | Gene Name | Gene Symbol |
|---|---|---|---|---|
| 1 | TOP Marker | 4775 | nuclear factor of activated T-cells, cytoplasmic, calcineurin-dependent 3 | NFATC3 |
| 2 | TOP Marker | 5982 | replication factor C (activator 1) 2, 40kDa | RFC2 |
| 3 | TOP Marker | - | Homo sapiens cDNA clone IMAGE:30377818 5'mRNA sequence | - |
| 4 | TOP Marker | 64129 | tubulointerstitial nephritis antigen-like 1 | TINAGL1 |
| 5 | TOP Marker | 10436 | EMG1 nucleolar protein homolog (S. cerevisiae) | EMG1 |
| 6 | TOP Marker | 6434 | transformer 2 beta homolog (Drosophila) | TRA2B |
| 7 | TOP Marker | 65109 | UPF3 regulator of nonsense transcripts homolog B (yeast) | UPF3B |
| 8 | TOP Marker | 6152 | ribosomal protein L24 | RPL24 |
| 9 | TOP Marker | 6838 | surfeit 6 | SURF6 |
| 10 | TOP Marker | 23608 | makorin ring finger protein 1 | MKRN1 |
| 11 | TOP Marker | 4155 | myelin basic protein | MBP |
| 12 | TOP Marker | 1938 | eukaryotic translation elongation factor 2 | EEF2 |
| 13 | TOP Marker | 27344 | proprotein convertase subtilisin/kexin type 1 inhibitor | PCSK1N |
| 14 | TOP Marker | - | OOF4155 | - |
| 15 | TOP Marker | - | OOF1938 | - |
| 16 | TOP Marker | - | OOF27344 | - |
| 17 | Marker | 58506 | SR-related CTD-associated factor 1 | SCAF1 |
| 18 | Marker | 324 | adenomatous polyposis coli | APC |
| 19 | Marker | 90861 | hematological and neurological expressed 1-like | HN1L |
| 20 | Marker | 119032 | chromosome 10 open reading frame 32 | C10orf32 |
| 21 | Marker | 84893 | F-box protein, helicase, 18 | FBXO18 |
| 22 | Marker | 10569 | SLU7 splicing factor homolog (S. cerevisiae) | SLU7 |
| 23 | Marker | 57455 | REX1, RNA exonuclease 1 homolog (S. cerevisiae) | REXO1 |
| 24 | Marker | 6461 | Src homology 2 domain containing adaptor protein B | SHB |
| 25 | Marker | 79155 | TNFAIP3 interacting protein 2 | TNIP2 |
| 26 | Marker | 339230 | coiled-coil domain containing 137 | CCDC137 |
| 27 | Marker | 23646 | phospholipase D family, member 3 | PLD3 |
| 28 | Marker | 11019 | lipoic acid synthetase | LIAS |
| 29 | Marker | 6130 | ribosomal protein L7a | RPL7A |
| 30 | Marker | 5831 | pyrroline-5-carboxylate reductase 1 | PYCR1 |
| 31 | Marker | 4122 | mannosidase, alpha, class 2A, member 2 | MAN2A2 |
| 32 | Marker | 51510 | chromatin modifying protein 5 | CHMP5 |
| 33 | Marker | 375690 | WAS protein family homolog 5 pseudogene | WASH5P |
| 34 | Marker | 3068 | hepatoma-derived growth factor | HDGF |
| 35 | Marker | 128866 | chromatin modifying protein 4B | CHMP4B |
| 36 | Marker | 7416 | voltage-dependent anion channel 1 | VDAC1 |
| 37 | Marker | 2037 | erythrocyte membrane protein band 4.1-like 2 | EPB41L2 |
| 38 | Marker | 4924 | nucleobindin 1 | NUCB1 |
| 39 | Marker | 7431 | vimentin | VIM |
| 40 | Marker | 10081 | programmed cell death 7 | PDCD7 |
| 41 | Marker | - | OOF2037 | - |
| 42 | Marker | - | OOF4924 | - |
| 43 | Marker | - | OOF7431 | - |
| 44 | Marker | - | OOF10081 | - |

**Table 2: Markers for NMO identified by statistical analysis of MFI data from NMO vs. Healthy. The sequence of the markers can be obtained from the enclosed sequence listening.**

| SEQ ID No. | Marker Classification | GeneID | Gene name | Gene Symbol |
|---|---|---|---|---|
| 45 | TOP Marker | 25841 | ankyrin repeat and BTB (POZ) domain containing 2 | ABTB2 |
| 46 | TOP Marker | 627 | brain-derived neurotrophic factor | BDNF |
| 47 | TOP Marker | 58506 | SR-related CTD-associated factor 1 | SCAF1 |
| 48 | TOP Marker | 5982 | replication factor C (activator 1) 2, 40kDa | RFC2 |
| 49 | TOP Marker | 3915 | laminin, gamma 1 (formerly LAMB2) | LAMC1 |
| 50 | TOP Marker | 324 | adenomatous polyposis coli | APC |
| 51 | TOP Marker | 5819 | poliovirus receptor-related 2 (herpesvirus entry mediator B) | PVRL2 |
| 52 | TOP Marker | 57455 | REX1, RNA exonuclease 1 homolog (S. cerevisiae) | REXO1 |
| 53 | TOP Marker | 10436 | EMG1 nucleolar protein homolog (S. cerevisiae) | EMG1 |
| 54 | TOP Marker | 55827 | DDB1 and CUL4 associated factor 6 | DCAF6 |
| 55 | TOP Marker | 5831 | pyrroline-5-carboxylate reductase 1 | PYCR1 |
| 56 | TOP Marker | 10445 | microspherule protein 1 | MCRS1 |
| 57 | TOP Marker | 128866 | chromatin modifying protein 4B | CHMP4B |
| 58 | TOP Marker | 3320 | heat shock protein 90kDa alpha (cytosolic), class A member 1 | HSP90AA1 |
| 59 | TOP Marker | 23608 | makorin ring finger protein 1 | MKRN1 |
| 60 | TOP Marker | 5370 | pro-melanin-concentrating hormone-like 2, pseudogene | PMCHL2 |
| 61 | TOP Marker | 11054 | opioid growth factor receptor | OGFR |
| 62 | TOP Marker | - | OOF5370 | - |
| 63 | TOP Marker | - | OOF11054 | - |
| 64 | Marker | 57136 | chromosome 20 open reading frame 3 | C20orf3 |
| 65 | Marker | 10075 | HECT, UBA and WWE domain containing 1 | HUWE1 |
| 66 | Marker | - | Homo sapiens cDNA clone IMAGE:30377818 5'mRNA sequence | - |
| 67 | Marker | 4744 | neurofilament, heavy polypeptide | NEFH |
| 68 | Marker | 7204 | triple functional domain (PTPRF interacting) | TRIO |
| 69 | Marker | 2935 | G1 to S phase transition 1 | GSPT1 |
| 70 | Marker | 64129 | tubulointerstitial nephritis antigen-like 1 | TINAGL1 |
| 71 | Marker | 10539 | glutaredoxin 3 | GLRX3 |
| 72 | Marker | 5595 | mitogen-activated protein kinase 3 | MAPK3 |
| 73 | Marker | 80728 | Rho GTPase activating protein 39 | ARHGAP39 |
| 74 | Marker | 1270 | ciliary neurotrophic factor | CNTF |
| 75 | Marker | 4354 | membrane protein, palmitoylated 1, 55kDa | MPP1 |
| 76 | Marker | 23114 | neurofascin | NFASC |
| 77 | Marker | 8874 | Rho guanine nucleotide exchange factor (GEF) 7 | ARHGEF7 |
| 78 | Marker | 65109 | UPF3 regulator of nonsense transcripts homolog B (yeast) | UPF3B |
| 79 | Marker | 7316 | ubiquitin C | UBC |
| 80 | Marker | 5864 | RAB3A, member RAS oncogene family | RAB3A |
| 81 | Marker | 79582 | sperm associated antigen 16 | SPAG16 |
| 82 | Marker | 2037 | erythrocyte membrane protein band 4.1-like 2 | EPB41L2 |
| 83 | Marker | 283248 | Homo sapiens REST corepressor 2 (RCOR2), mRNA | RCOR2 |
| 84 | Marker | - | OOF5864 | - |
| 85 | Marker | - | OOF79582 | - |
| 86 | Marker | - | OOF2037 | - |
| 87 | Marker | - | OOF283248 | - |

### Univariate Analysis:

**Table 3.1 NMO vs healthy controls**

| | | | | | | | | | | | |
|---|---|---|---|---|---|---|---|---|---|---|---|
| Scoring and identification of univariate TOP 30 candidates based on score and p-value ranking. | | | | | | | | | | | |

| | Score | ProteinID | GeneID | Gene Name | p-value | Fold change | effect size | Fisher's ratio | Median 1 | Median 2 | AUC |
|---|---|---|---|---|---|---|---|---|---|---|---|
| 1 | 6,5 | BBA00.260_1043136934 | 361 | aquaporin 4 | 0,0020 | 7,46 | 1,81 | 1,49 | 303 | 2263 | 0,875 |
| 2 | 6,5 | BBA00.347_1043135290 | 11054 | opioid growth factor receptor | 0,0039 | 7,48 | 1,40 | 0,90 | 546 | 4085 | 0,851 |
| 3 | 6,5 | BBA00.288_1043143161 | 10436 | EMG1 nucleolar protein homolog (S. cerevisiae) | 0,0130 | 2,22 | 1,30 | 0,77 | 515 | 1144 | 0,802 |
| 4 | 6,5 | BBA00.315_1043143926 | 10445 | microspherule protein 1 | 0,0166 | -4,02 | 1,14 | 0,60 | 4368 | 1088 | 0,792 |
| 5 | 6,5 | BBA00.392_1043140856 | 23608 | makorin ring finger protein 1 | 0,0226 | 2,63 | 1,13 | 0,58 | 1191 | 3134 | 0,778 |
| 6 | 6,5 | BBA00.119_1043136825 | 324 | adenomatous polyposis coli | 0,0281 | 3,09 | 1,09 | 0,54 | 1116 | 3453 | 0,767 |
| 7 | 6,5 | BBA00.205_1043143644 | 57455 | REX1, RNA exonuclease 1 homolog (S. cerevisiae) | 0,0304 | 3,21 | 1,07 | 0,52 | 332 | 1065 | 0,764 |
| 8 | 6 | BBA00.365_1043144408 | 3320 | heat shock protein 90kDa alpha (cytosolic), class A member 1 | 0,0011 | 2,13 | 1,57 | 1,13 | 237 | 506 | 0,896 |
| 9 | 6 | BBA00.298_1043143832 | 55827 | DDB1 and CUL4 associated factor 6 | 0,0225 | 3,69 | 1,04 | 0,49 | 147 | 544 | 0,778 |
| 10 | 6 | BBA00.044_1043136937 | 25841 | ankyrin repeat and BTB (POZ) domain containing 2 | 0,0351 | -2,41 | 1,05 | 0,50 | 510 | 211 | 0,757 |
| 11 | 5,5 | BBA00.272_1043143163 | No Gene ID | NA | 0,0783 | -2,09 | 0,56 | 0,15 | 1452 | 694 | 0,715 |
| 12 | 5,5 | BBA00.198_1043136831 | 2935 | G1 to S phase transition 1 | 0,0831 | 2,78 | 0,75 | 0,26 | 2659 | 7402 | 0,712 |
| 13 | 5,5 | BBA00.231_1043135293 | 10539 | glutaredoxin 3 | 0,1409 | -3,98 | 0,59 | 0,16 | 5562 | 1398 | 0,681 |
| 14 | 5 | BBA00.075_1043137831 | 5370 | pro-melanin-concentrating hormone-like 2, pseudogene | 0,0035 | -2,04 | 1,27 | 0,74 | 149 | 73 | 0,854 |
| 15 | 5 | BBA00.311_1043143356 | 5831 | pyrroline-5-carboxylate reductase 1 | 0,0079 | 2,67 | 0,84 | 0,32 | 129 | 343 | 0,823 |
| 16 | 5 | BBA00.210_1043140481 | 64129 | tubulointerstitial nephritis antigen-like 1 | 0,0120 | -1,81 | 1,05 | 0,50 | 652 | 360 | 0,806 |
| 17 | 5 | BBA00.351_1043144220 | 128866 | chromatin modifying protein 4B | 0,0120 | 2,23 | 0,79 | 0,28 | 159 | 354 | 0,806 |
| 18 | 5 | BBA00.068_1043138278 | 5982 | replication factor C (activator 1) 2, 40kDa | 0,0130 | -2,55 | 1,04 | 0,50 | 297 | 117 | 0,802 |
| 19 | 5 | BBA00.047_1043136648 | 627 | brain-derived neurotrophic factor | 0,0194 | -2,08 | 1,19 | 0,65 | 136 | 65 | 0,785 |
| 20 | 5 | BBA00.150_1043141334 | 7204 | triple functional domain (PTPRF interacting) | 0,0304 | -1,99 | 0,55 | 0,14 | 700 | 352 | 0,764 |
| 21 | 5 | BBA00.251_1043141343 | 80728 | Rho GTPase activating protein 39 | 0,0304 | -1,70 | 1,03 | 0,49 | 785 | 461 | 0,764 |
| 22 | 5 | BBA00.109_1043137791 | 3915 | laminin, gamma 1 (formerly LAMB2) | 0,0404 | 2,42 | 0,99 | 0,45 | 355 | 858 | 0,750 |
| 23 | 5 | BBA00.140_1043143542 | 5819 | poliovirus receptor-related 2 (herpesvirus entry mediator B) | 0,0404 | 3,84 | 0,96 | 0,42 | 183 | 701 | 0,750 |
| 24 | 5 | BBA00.059_1043138765 | 58506 | SR-related CTD-associated factor 1 | 0,0464 | -3,13 | 0,96 | 0,42 | 774 | 248 | 0,743 |
| 25 | 5 | BBA00.237_1043135291 | 5595 | mitogen-activated protein kinase 3 | 0,0781 | -2,02 | 0,85 | 0,33 | 653 | 322 | 0,715 |
| 26 | 5 | BBA00.080_1043143745 | No Gene ID | NA | 0,1058 | 3,58 | 0,61 | 0,17 | 145 | 520 | 0,698 |
| 27 | 5 | BBA00.284_1043136634 | 1270 | ciliary neurotrophic factor | 0,1124 | -2,06 | 0,51 | 0,12 | 887 | 430 | 0,694 |
| 28 | 4,5 | BBA00.320_1043140474 | 8874 | Rho guanine nucleotide exchange factor (GEF) 7 | 0,0646 | 1,46 | 0,59 | 0,16 | 758 | 1110 | 0,726 |
| 29 | 4,5 | BBA00.036_1043143937 | 5864 | RAB3A, member RAS oncogene family | 0,0831 | -1,78 | 0,99 | 0,45 | 1268 | 713 | 0,712 |
| 30 | 4,5 | BBA00.340_1043138937 | 65109 | UPF3 regulator of nonsense transcripts homolog B (yeast) | 0,0997 | 1,90 | 0,96 | 0,43 | 1200 | 2286 | 0,701 |

**Table 3.2 NMO vs MS**

| | | | | | | | | | | | |
|---|---|---|---|---|---|---|---|---|---|---|---|
| Scoring and identification of univariate TOP 30 candidates based on score and p-value ranking. | | | | | | | | | | | |

| | Score | ProteinID | GeneID | Gene Name | p-value | Fold change | effect size | Fisher's ratio | Median 1 | Median 2 | AUC |
|---|---|---|---|---|---|---|---|---|---|---|---|
| 1 | 6,5 | BBA00.260_1043136934 | 361 | aquaporin 4 | 0,0007 | 8,91 | 1,71 | 1,37 | 254 | 2263 | 0,875 |
| 2 | 6,5 | BBA00.288_1043143161 | 10436 | EMG1 nucleolar protein homolog (S. cerevisiae) | 0,0072 | 2,37 | 0,60 | 0,19 | 482 | 1144 | 0,796 |
| 3 | 6,5 | BBA00.340_1043138937 | 65109 | UPF3 regulator of nonsense transcripts homolog B (yeast) | 0,0209 | 2,20 | 0,55 | 0,16 | 1038 | 2286 | 0,755 |
| 4 | 6 | BBA00.210_1043140481 | 64129 | tubulointerstitial nephritis antigen-like 1 | 0,0033 | -2,08 | 1,27 | 0,78 | 748 | 360 | 0,824 |
| 5 | 6 | BBA00.035_1043138758 | 4775 | nuclear factor of activated T-cells, cytoplasmic, calcineurin-dependent 3 | 0,0158 | 2,77 | 1,00 | 0,44 | 314 | 870 | 0,766 |
| 6 | 5,5 | BBA00.324_1043137810 | 27344 | proprotein convertase subtilisin/kexin type 1 inhibitor | 0,0246 | -7,44 | 1,03 | 0,49 | 16628 | 2235 | 0,748 |
| 7 | 5,5 | BBA00.376_1043143543 | 6838 | surfeit 6 | 0,0308 | -2,39 | 0,76 | 0,27 | 1258 | 526 | 0,738 |
| 8 | 5,5 | BBA00.295_1043138939 | 1938 | eukaryotic translation elongation factor 2 | 0,0380 | 3,92 | 0,94 | 0,40 | 320 | 1253 | 0,729 |
| 9 | 5,5 | BBA00.343_1043143350 | 6152 | ribosomal protein L24 | 0,0443 | -2,14 | 0,91 | 0,41 | 1007 | 471 | 0,722 |
| 10 | 5,5 | BBA00.392_1043140856 | 23608 | makorin ring finger protein 1 | 0,0443 | 2,71 | 0,85 | 0,33 | 1156 | 3134 | 0,722 |
| 11 | 5,5 | BBA00.328_1043140473 | 6434 | transformer 2 beta homolog (Drosophila) | 0,0466 | -3,14 | 0,71 | 0,23 | 1773 | 565 | 0,720 |
| 12 | 5,5 | BBA00.354_1043142586 | 7431 | vimentin | 0,0655 | 3,50 | 0,80 | 0,28 | 319 | 1117 | 0,704 |
| 13 | 5,5 | BBA00.333_1043144502 | 375690 | WAS protein family homolog 5 pseudogene | 0,0789 | 2,05 | 0,69 | 0,23 | 1851 | 3801 | 0,694 |
| 14 | 5,5 | BBA00.213_1043140091 | 6461 | Src homology 2 domain containing adaptor protein B | 0,1892 | 2,12 | 0,43 | 0,09 | 1220 | 2591 | 0,646 |
| 15 | 5,5 | BBA00.205_1043143644 | 57455 | REX1, RNA exonuclease 1 homolog (S. cerevisiae) | 0,2040 | 2,21 | 0,55 | 0,13 | 482 | 1065 | 0,641 |
| 16 | 5,5 | BBA00.119_1043136825 | 324 | adenomatous polyposis coli | 0,2116 | 3,17 | 0,64 | 0,18 | 1089 | 3453 | 0,639 |
| 17 | 5 | BBA00.302_1043135287 | 6130 | ribosomal protein L7a | 0,0017 | -1,70 | 1,09 | 0,64 | 605 | 355 | 0,845 |
| 18 | 5 | BBA00.252_1043144317 | 339230 | coiled-coil domain containing 137 | 0,0025 | -1,53 | 1,02 | 0,54 | 764 | 501 | 0,833 |
| 19 | 5 | BBA00.027_1043138757 | 4155 | myelin basic protein | 0,0052 | 2,27 | 0,95 | 0,41 | 197 | 449 | 0,808 |
| 20 | 5 | BBA00.317_1043144508 | 4122 | mannosidase, alpha, class 2A, member 2 | 0,0092 | -1,97 | 0,96 | 0,46 | 844 | 428 | 0,787 |
| 21 | 5 | BBA00.068_1043138278 | 5982 | replication factor C (activator 1) 2,40kDa | 0,0092 | -3,42 | 1,13 | 0,62 | 398 | 117 | 0,787 |
| 22 | 5 | BBA00.263_1043138267 | 23646 | phospholipase D family, member 3 | 0,0098 | -1,57 | 1,25 | 0,78 | 693 | 441 | 0,785 |
| 23 | 5 | BBA00.242_0105509577 | 79155 | TNFAIP3 interacting protein 2 | 0,0199 | -1,80 | 1,01 | 0,52 | 891 | 496 | 0,757 |
| 24 | 5 | BBA00.193_1043143261 | 10569 | SLU7 splicing factor homolog (S. cerevisiae) | 0,0210 | -1,76 | 0,67 | 0,23 | 737 | 418 | 0,755 |
| 25 | 5 | BBA00.080_1043143745 | No Gene ID | NA | 0,0262 | 3,80 | 0,99 | 0,44 | 137 | 520 | 0,745 |
| 26 | 5 | BBA00.059_1043138765 | 58506 | SR-related CTD-associated factor 1 | 0,0541 | -2,19 | 0,83 | 0,32 | 542 | 248 | 0,713 |
| 27 | 5 | BBA00.189_1043143648 | 84893 | F-box protein, helicase, 18 | 0,0541 | 3,45 | 0,82 | 0,30 | 218 | 752 | 0,713 |
| 28 | 5 | BBA00.169_1043144025 | 90861 | hematological and neurological expressed 1-like | 0,0987 | 2,32 | 0,66 | 0,19 | 241 | 559 | 0,683 |
| 29 | 4,5 | BBA00.400_1043140097 | 10081 | programmed cell death 7 | 0,0325 | -1,99 | 0,89 | 0,36 | 1485 | 745 | 0,736 |
| 30 | 4,5 | BBA00.280_1043143162 | 11019 | lipoic acid synthetase | 0,0325 | 1,56 | 1,00 | 0,41 | 754 | 1179 | 0,736 |

**Table 3.3 NMO vs healthy controls**

| | | | | | | | | | | | |
|---|---|---|---|---|---|---|---|---|---|---|---|
| "Edge candidates" resulting from Volcano plot, fold-change at least 2 in either direction and p-value <0.05. | | | | | | | | | | | |

| | ProteinID | GeneID | Gene Name | p-value | adjusted p-value | test statistic | fold-change | FDR | effect size | log2 (ratio) | -log10 (p-value) |
|---|---|---|---|---|---|---|---|---|---|---|---|
| 1 | BBA00.365_1043144408 | 3320 | heat shock protein 90kDa alpha (cytosolic), class A member 1 | 0,0011 | 0,2708 | 15 | 2,13 | 0,14 | 1,57 | 1,09 | 2,96 |
| 2 | BBA00.047_1043136648 | 627 | brain-derived neurotrophic factor | 0,0194 | 1,0000 | 113 | -2,08 | 0,32 | 1,19 | -1,06 | 1,71 |
| 3 | BBA00.298_1043143832 | 55827 | DDB1 and CUL4 associated factor 6 | 0,0225 | 1,0000 | 32 | 3,69 | 0,32 | 1,04 | 1,89 | 1,65 |
| 4 | BBA00.392_1043140856 | 23608 | makorin ring finger protein 1 | 0,0226 | 1,0000 | 32 | 2,63 | 0,32 | 1,13 | 1,40 | 1,65 |
| 5 | BBA00.119_1043136825 | 324 | adenomatous polyposis coli | 0,0281 | 1,0000 | 33,5 | 3,09 | 0,32 | 1,09 | 1,63 | 1,55 |
| 6 | BBA00.205_1043143644 | 57455 | REX1, RNA exonuclease 1 homolog (S. cerevisiae) | 0,0304 | 1,0000 | 34 | 3,21 | 0,32 | 1,07 | 1,68 | 1,52 |
| 7 | BBA00.044_1043136937 | 25841 | ankyrin repeat and BTB (POZ) domain containing 2 | 0,0351 | 1,0000 | 109 | -2,41 | 0,32 | 1,05 | -1,27 | 1,45 |
| 8 | BBA00.109_1043137791 | 3915 | laminin, gamma 1 (formerly LAMB2) | 0,0404 | 1,0000 | 36 | 2,42 | 0,32 | 0,99 | 1,27 | 1,39 |
| 9 | BBA00.140_1043143542 | 5819 | poliovirus receptor-related 2 (herpesvirus entry mediator B) | 0,0404 | 1,0000 | 36 | 3,84 | 0,32 | 0,96 | 1,94 | 1,39 |
| 10 | BBA00.059_1043138765 | 58506 | SR-related CTD-associated factor 1 | 0,0464 | 1,0000 | 107 | -3,13 | 0,34 | 0,96 | -1,64 | 1,33 |
| 11 | BBA00.260_1043136934 | 361 | aquaporin 4 | 0,0020 | 0,4923 | 18 | 7,46 | 0,17 | 1,81 | 2,90 | 2,70 |
| 12 | BBA00.075_1043137831 | 5370 | pro-melanin-concentrating hormone-like 2, pseudogene | 0,0035 | 0,8626 | 123 | -2,04 | 0,19 | 1,27 | -1,03 | 2,45 |
| 13 | BBA00.347_1043135290 | 11054 | opioid growth factor receptor | 0,0039 | 0,9421 | 21,5 | 7,48 | 0,19 | 1,40 | 2,90 | 2,41 |
| 14 | BBA00.311_1043143356 | 5831 | pyrroline-5-carboxylate reductase 1 | 0,0079 | 1,0000 | 25,5 | 2,67 | 0,27 | 0,84 | 1,42 | 2,10 |
| 15 | BBA00.351_1043144220 | 128866 | chromatin modifying protein 4B | 0,0120 | 1,0000 | 28 | 2,23 | 0,27 | 0,79 | 1,16 | 1,92 |
| 16 | BBA00.068_1043138278 | 5982 | replication factor C (activator 1) 2, 40kDa | 0,0130 | 1,0000 | 115,5 | -2,55 | 0,27 | 1,04 | -1,35 | 1,89 |
| 17 | BBA00.288_1043143161 | 10436 | EMG1 nucleolar protein homolog (S. cerevisiae) | 0,0130 | 1,0000 | 28,5 | 2,22 | 0,27 | 1,30 | 1,15 | 1,89 |
| 18 | BBA00.315_1043143926 | 10445 | microspherule protein 1 | 0,0166 | 1,0000 | 114 | -4,02 | 0,29 | 1,14 | -2,01 | 1,78 |

**Table 3.4 NMO vs MS**

| | | | | | | | | | | | |
|---|---|---|---|---|---|---|---|---|---|---|---|
| "Edge candidates" resulting from Volcano plot, fold-change at least 2 in either direction and p-value <0.05. | | | | | | | | | | | |

| | ProteinID | GeneID | Gene Name | p-value | adjusted p-value | test statistic | fold-change | FDR | effect size | log2 (ratio) | -log10 (p-value) |
|---|---|---|---|---|---|---|---|---|---|---|---|
| 1 | BBA00.260_1043136934 | 361 | aquaporin 4 | 0,0007 | 0,1611 | 27,00 | 8,91 | 0,08 | 1,71 | 3,16 | 3,18 |
| 2 | BBA00.376_1043143543 | 6838 | surfeit 6 | 0,0308 | 1,0000 | 159,50 | -2,39 | 0,33 | 0,76 | -1,26 | 1,51 |
| 3 | BBA00.295_1043138939 | 1938 | eukaryotic translation elongation factor 2 | 0,0380 | 1,0000 | 58,50 | 3,92 | 0,33 | 0,94 | 1,97 | 1,42 |
| 4 | BBA00.343_1043143350 | 6152 | ribosomal protein L24 | 0,0443 | 1,0000 | 156,00 | -2,14 | 0,33 | 0,91 | -1,10 | 1,35 |
| 5 | BBA00.392_1043140856 | 23608 | makorin ring finger protein 1 | 0,0443 | 1,0000 | 60,00 | 2,71 | 0,33 | 0,85 | 1,44 | 1,35 |
| 6 | BBA00.328_1043140473 | 6434 | transformer 2 beta homolog (Drosophila) | 0,0466 | 1,0000 | 155,50 | -3,14 | 0,34 | 0,71 | -1,65 | 1,33 |
| 7 | BBA00.210_1043140481 | 64129 | tubulointerstitial nephritis antigen-like 1 | 0,0033 | 0,7838 | 178,00 | -2,08 | 0,11 | 1,27 | -1,05 | 2,49 |
| 8 | BBA00.027_1043138757 | 4155 | myelin basic protein | 0,0052 | 1,0000 | 41,50 | 2,27 | 0,16 | 0,95 | 1,18 | 2,28 |
| 9 | BBA00.288_1043143161 | 10436 | EMG1 nucleolar protein homolog (S. cerevisiae) | 0,0072 | 1,0000 | 44,00 | 2,37 | 0,19 | 0,60 | 1,25 | 2,14 |
| 10 | BBA00.068_1043138278 | 5982 | replication factor C (activator 1) 2, 40kDa | 0,0092 | 1,0000 | 170,00 | -3,42 | 0,19 | 1,13 | -1,77 | 2,04 |
| 11 | BBA00.035_1043138758 | 4775 | nuclear factor of activated T-cells, cytoplasmic, calcineurin-dependent 3 | 0,0158 | 1,0000 | 50,50 | 2,77 | 0,26 | 1,00 | 1,47 | 1,80 |
| 12 | BBA00.340_1043138937 | 65109 | UPF3 regulator of nonsense transcripts homolog B (yeast) | 0,0209 | 1,0000 | 53,00 | 2,20 | 0,27 | 0,55 | 1,14 | 1,68 |
| 13 | BBA00.324_1043137810 | 27344 | proprotein convertase subtilisin/kexin type 1 inhibitor | 0,0246 | 1,0000 | 161,50 | -7,44 | 0,30 | 1,03 | -2,90 | 1,61 |
| 14 | BBA00.080_1043143745 | No Gene ID | NA | 0,0262 | 1,0000 | 55,00 | 3,80 | 0,31 | 0,99 | 1,93 | 1,58 |

### Multivariate Analysis:

**Table 4.1 NMO vs healthy controls**

| | | | | | |
|---|---|---|---|---|---|
| Antigens with freq >/= 100 or (freq >/=80 and median rank </= 16) obtained from a ranking list and panel definition according to PPLS-DA TOP 30 (200 runs) based on all antigens. | | | | | |

| | ProteinID | GeneID | Gene.Name | freq | median rank |
|---|---|---|---|---|---|
| 16 | BBA00.260_1043136934 | 361 | aquaporin 4 | 200 | 1 |
| 25 | BBA00.347_1043135290 | 11054 | opioid growth factor receptor | 200 | 3 |
| 22 | BBA00.315_1043143926 | 10445 | microspherule protein 1 | 199 | 5 |
| 27 | BBA00.392_1043140856 | 23608 | makorin ring finger protein 1 | 192 | 6 |
| 14 | BBA00.205_1043143644 | 57455 | REX1, RNA exonuclease 1 homolog (S. cerevisiae) | 187 | 11 |
| 4 | BBA00.059_1043138765 | 58506 | SR-related CTD-associated factor 1 | 182 | 8 |
| 10 | BBA00.119_1043136825 | 324 | adenomatous polyposis coli | 179 | 14 |
| 19 | BBA00.298_1043143832 | 55827 | DDB1 and CUL4 associated factor 6 | 163 | 11 |
| 12 | BBA00.140_1043143542 | 5819 | poliovirus receptor-related 2 (herpesvirus entry mediator B) | 161 | 13 |
| 2 | BBA00.036_1043143937 | 5864 | RAB3A, member RAS oncogene family | 160 | 7 |
| 9 | BBA00.109_1043137791 | 3915 | laminin, gamma 1 (formerly LAMB2) | 155 | 13,5 |
| 7 | BBA00.068_1043138278 | 5982 | replication factor C (activator 1) 2, 40kDa | 149 | 15 |
| 15 | BBA00.251_1043141343 | 80728 | Rho GTPase activating protein 39 | 142 | 18 |
| 26 | BBA00.365_1043144408 | 3320 | heat shock protein 90kDa alpha (cytosolic), class A member 1 | 142 | 7 |
| 23 | BBA00.340_1043138937 | 65109 | UPF3 regulator of nonsense transcripts homolog B (yeast) | 132 | 17,5 |
| 6 | BBA00.066_1043142796 | 10075 | HECT, UBA and WWE domain containing 1 | 126 | 15 |
| 11 | BBA00.134_1043141344 | 4744 | neurofilament, heavy polypeptide | 125 | 15 |
| 5 | BBA00.065_1043140868 | 79582 | sperm associated antigen 16 | 123 | 19 |
| 8 | BBA00.075_1043137831 | 5370 | pro-melanin-concentrating hormone-like 2, pseudogene | 113 | 13 |
| 20 | BBA00.308_1043136631 | 23114 | neurofascin | 113 | 17 |
| 1 | BBA00.032_1043143271 | 57136 | chromosome 20 open reading frame 3 | 109 | 19 |
| 17 | BBA00.286_1043143930 | 4354 | membrane protein, palmitoylated 1,55kDa | 104 | 20 |
| 21 | BBA00.311_1043143356 | 5831 | pyrroline-5-carboxylate reductase 1 | 100 | 18 |
| 24 | BBA00.341_1043144416 | 7316 | ubiquitin C | 100 | 21 |
| 13 | BBA00.198_1043136831 | 2935 | G1 to S phase transition 1 | 99 | 16 |
| 3 | BBA00.047_1043136648 | 627 | brain-derived neurotrophic factor | 98 | 15 |
| 18 | BBA00.288_1043143161 | 10436 | EMG1 nucleolar protein homolog (S. cerevisiae) | 95 | 15 |

**Table 4.2 NMO vs healthy controls**

| | | | | |
|---|---|---|---|---|
| Ranking list of PPLS-DA results based on antigens with freq >/= 100 or (freq >/= 80 and median rank </= 16) obtained from a ranking list and panel definition according to PPLS-DA TOP 30 (200 runs) based on all antigens. | | | | |

| | ProteinID | GeneID | Gene.Name | abs.loading.weight.comp.1. |
|---|---|---|---|---|
| x.BBA00.260_1043136934 | BBA00.260_1043136934 | 361 | aquaporin 4 | 0,39 |
| x.BBA00.347_1043135290 | BBA00.347_1043135290 | 11054 | opioid growth factor receptor | 0,31 |
| x.BBA00.315_1043143926 | BBA00.315_1043143926 | 10445 | microspherule protein 1 | 0,24 |
| x.BBA00.392_1043140856 | BBA00.392_1043140856 | 23608 | makorin ring finger protein 1 | 0,23 |
| x.BBA00.365_1043144408 | BBA00.365_1043144408 | 3320 | heat shock protein 90kDa alpha (cytosolic), class A member 1 | 0,20 |
| x.BBA00.059_1043138765 | BBA00.059_1043138765 | 58506 | SR-related CTD-associated factor 1 | 0,20 |
| x.BBA00.036_1043143937 | BBA00.036_1043143937 | 5864 | RAB3A, member RAS oncogene family | 0,20 |
| x.BBA00.205_1043143644 | BBA00.205_1043143644 | 57455 | REX1, RNA exonuclease 1 homolog (S. cerevisiae) | 0,19 |
| x.BBA00.119_1043136825 | BBA00.119_1043136825 | 324 | adenomatous polyposis coli | 0,19 |
| x.BBA00.298_1043143832 | BBA00.298_1043143832 | 55827 | DDB1 and CUL4 associated factor 6 | 0,18 |
| x.BBA00.140_1043143542 | BBA00.140_1043143542 | 5819 | poliovirus receptor-related 2 (herpesvirus entry mediator B) | 0,17 |
| x.BBA00.109_1043137791 | BBA00.109_1043137791 | 3915 | laminin, gamma 1 (formerly LAMB2) | 0,17 |
| x.BBA00.068_1043138278 | BBA00.068_1043138278 | 5982 | replication factor C (activator 1) 2, 40kDa | 0,17 |
| x.BBA00.075_1043137831 | BBA00.075_1043137831 | 5370 | pro-melanin-concentrating hormone-like 2, pseudogene | 0,16 |
| x.BBA00.251_1043141343 | BBA00.251_1043141343 | 80728 | Rho GTPase activating protein 39 | 0,16 |
| x.BBA00.134_1043141344 | BBA00.134_1043141344 | 4744 | neurofilament, heavy polypeptide | 0,16 |
| x.BBA00.065_1043140868 | BBA00.065_1043140868 | 79582 | sperm associated antigen 16 | 0,16 |
| x.BBA00.288_1043143161 | BBA00.288_1043143161 | 10436 | EMG1 nucleolar protein homolog (S. cerevisiae) | 0,16 |
| x.BBA00.066_1043142796 | BBA00.066_1043142796 | 10075 | HECT, UBA and WWE domain containing 1 | 0,16 |
| x.BBA00.340_1043138937 | BBA00.340_1043138937 | 65109 | UPF3 regulator of nonsense transcripts homolog B (yeast) | 0,16 |
| x.BBA00.032_1043143271 | BBA00.032_1043143271 | 57136 | chromosome 20 open reading frame 3 | 0,16 |
| x.BBA00.047_1043136648 | BBA00.047_1043136648 | 627 | brain-derived neurotrophic factor | 0,15 |
| x.BBA00.311_1043143356 | BBA00.311_1043143356 | 5831 | pyrroline-5-carboxylate reductase 1 | 0,14 |
| x.BBA00.308_1043136631 | BBA00.308_1043136631 | 23114 | neurofascin | 0,14 |
| x.BBA00.341_1043144416 | BBA00.341_1043144416 | 7316 | ubiquitin C | 0,14 |
| x.BBA00.198_1043136831 | BBA00.198_1043136831 | 2935 | G1 to S phase transition 1 | 0,13 |
| x.BBA00.286_1043143930 | BBA00.286_1043143930 | 4354 | membrane protein, palmitoylated 1, 55kDa | 0,13 |

**Table 4.3. NMO vs healthy controls**

| | | | | | |
|---|---|---|---|---|---|
| Antigens from ranking list and panel definition according to PPLS-DA TOP 30 (200 runs) based on all antigens without AQP-4 and with freq >/= 100 or (freq >/= 80 and median rank </= 16). | | | | | |

| | ProteinID | GeneID | Gene.Name | freq | median rank |
|---|---|---|---|---|---|
| 25 | BBA00.347_1043135290 | 11054 | opioid growth factor receptor | 200 | 2 |
| 22 | BBA00.315_1043143926 | 10445 | microspherule protein 1 | 193 | 4 |
| 27 | BBA00.392_1043140856 | 23608 | makorin ring finger protein 1 | 191 | 5 |
| 16 | BBA00.205_1043143644 | 57455 | REX1, RNA exonuclease 1 homolog (S. cerevisiae) | 188 | 10 |
| 11 | BBA00.119_1043136825 | 324 | adenomatous polyposis coli | 178 | 12 |
| 5 | BBA00.059_1043138765 | 58506 | SR-related CTD-associated factor 1 | 175 | 8 |
| 19 | BBA00.298_1043143832 | 55827 | DDB1 and CUL4 associated factor 6 | 164 | 11 |
| 10 | BBA00.109_1043137791 | 3915 | laminin, gamma 1 (formerly LAMB2) | 162 | 14 |
| 13 | BBA00.140_1043143542 | 5819 | poliovirus receptor-related 2 (herpesvirus entry mediator B) | 162 | 12 |
| 2 | BBA00.036_1043143937 | 5864 | RAB3A, member RAS oncogene family | 161 | 7 |
| 17 | BBA00.251_1043141343 | 80728 | Rho GTPase activating protein 39 | 156 | 16 |
| 8 | BBA00.068_1043138278 | 5982 | replication factor C (activator 1) 2, 40kDa | 153 | 13 |
| 26 | BBA00.365_1043144408 | 3320 | heat shock protein 90kDa alpha (cytosolic), class A member 1 | 144 | 5 |
| 23 | BBA00.340_1043138937 | 65109 | UPF3 regulator of nonsense transcripts homolog B (yeast) | 138 | 16 |
| 1 | BBA00.032_1043143271 | 57136 | chromosome 20 open reading frame 3 | 134 | 18 |
| 6 | BBA00.065_1043140868 | 79582 | sperm associated antigen 16 | 133 | 16,5 |
| 9 | BBA00.075_1043137831 | 5370 | pro-melanin-concentrating hormone-like 2, pseudogene | 127 | 11,5 |
| 7 | BBA00.066_1043142796 | 10075 | HECT, UBA and WWE domain containing 1 | 125 | 15 |
| 12 | BBA00.134_1043141344 | 4744 | neurofilament, heavy polypeptide | 115 | 13 |
| 15 | BBA00.201_1043143257 | 2037 | erythrocyte membrane protein band 4.1-like 2 | 115 | 18 |
| 20 | BBA00.308_1043136631 | 23114 | neurofascin | 112 | 17 |
| 24 | BBA00.341_1043144416 | 7316 | ubiquitin C | 108 | 20 |
| 18 | BBA00.288_1043143161 | 10436 | EMG1 nucleolar protein homolog (S. cerevisiae) | 102 | 12 |
| 4 | BBA00.047_1043136648 | 627 | brain-derived neurotrophic factor | 99 | 13 |
| 14 | BBA00.198_1043136831 | 2935 | G1 to S phase transition 1 | 98 | 14,5 |
| 21 | BBA00.311_1043143356 | 5831 | pyrroline-5-carboxylate reductase 1 | 93 | 15 |
| 3 | BBA00.044_1043136937 | 25841 | ankyrin repeat and BTB (POZ) domain containing 2 | 82 | 15 |

**Table 4.4 NMO vs healthy controls**

| | | | | |
|---|---|---|---|---|
| Ranking list of PPLS-DA results (TOP 30 antigens based on 200 runs) based on antigens without AQP-4 and with freq > / = 100 or (freq >/= 80 and median rank </= 16). | | | | |

| | ProteinID | GeneID | Gene.Name | abs.loading.weight.comp.1. |
|---|---|---|---|---|
| x.BBA00.347_1043135290 | BBA00.347_1043135290 | 11054 | opioid growth factor receptor | 0,34087 |
| x.BBA00.365_1043144408 | BBA00.365_1043144408 | 3320 | heat shock protein 90kDa alpha (cytosolic), class A member 1 | 0,26728 |
| x.BBA00.315_1043143926 | BBA00.315_1043143926 | 10445 | microspherule protein 1 | 0,24583 |
| x.BBA00.392_1043140856 | BBA00.392_1043140856 | 23608 | makorin ring finger protein 1 | 0,24173 |
| x.BBA00.205_1043143644 | BBA00.205_1043143644 | 57455 | REX1, RNA exonuclease 1 homolog (S. cerevisiae) | 0,20385 |
| x.BBA00.119_1043136825 | BBA00.119_1043136825 | 324 | adenomatous polyposis coli | 0,20278 |
| x.BBA00.075_1043137831 | BBA00.075_1043137831 | 5370 | pro-melanin-concentrating hormone-like 2, pseudogene | 0,20127 |
| x.BBA00.036_1043143937 | BBA00.036_1043143937 | 5864 | RAB3A, member RAS oncogene family | 0,19844 |
| x.BBA00.288_1043143161 | BBA00.288_1043143161 | 10436 | EMG1 nucleolar protein homolog (S. cerevisiae) | 0,19741 |
| x.BBA00.059_1043138765 | BBA00.059_1043138765 | 58506 | SR-related CTD-associated factor 1 | 0,19537 |
| x.BBA00.298_1043143832 | BBA00.298_1043143832 | 55827 | DDB1 and CUL4 associated factor 6 | 0,19032 |
| x.BBA00.065_1043140868 | BBA00.065_1043140868 | 79582 | sperm associated antigen 16 | 0,18421 |
| x.BBA00.068_1043138278 | BBA00.068_1043138278 | 5982 | replication factor C (activator 1) 2, 40kDa | 0,18361 |
| x.BBA00.047_1043136648 | BBA00.047_1043136648 | 627 | brain-derived neurotrophic factor | 0,18254 |
| x.BBA00.032_1043143271 | BBA00.032_1043143271 | 57136 | chromosome 20 open reading frame 3 | 0,18069 |
| x.BBA00.109_1043137791 | BBA00.109_1043137791 | 3915 | laminin, gamma 1 (formerly LAMB2) | 0,17799 |
| x.BBA00.140_1043143542 | BBA00.140_1043143542 | 5819 | poliovirus receptor-related 2 (herpesvirus entry mediator B) | 0,17400 |
| x.BBA00.251_1043141343 | BBA00.251_1043141343 | 80728 | Rho GTPase activating protein 39 | 0,17348 |
| x.BBA00.201_1043143257 | BBA00.201_1043143257 | 2037 | erythrocyte membrane protein band 4.1-like 2 | 0,16699 |
| x.BBA00.340_1043138937 | BBA00.340_1043138937 | 65109 | UPF3 regulator of nonsense transcripts homolog B (yeast) | 0,16068 |
| x.BBA00.044_1043136937 | BBA00.044_1043136937 | 25841 | ankyrin repeat and BTB (POZ) domain containing 2 | 0,15194 |
| x.BBA00.066_1043142796 | BBA00.066_1043142796 | 10075 | HECT, UBA and WWE domain containing 1 | 0,15067 |
| x.BBA00.134_1043141344 | BBA00.134_1043141344 | 4744 | neurofilament, heavy polypeptide | 0,14964 |
| x.BBA00.341_1043144416 | BBA00.341_1043144416 | 7316 | ubiquitin C | 0,13914 |
| x.BBA00.311_1043143356 | BBA00.311_1043143356 | 5831 | pyrroline-5-carboxylate reductase 1 | 0,13672 |
| x.BBA00.308_1043136631 | BBA00.308_1043136631 | 23114 | neurofascin | 0,13444 |
| x.BBA00.198_1043136831 | BBA00.198_1043136831 | 2935 | G1 to S phase transition 1 | 0,11788 |

**Table 4.5 NMO vs MS**

| | | | | | |
|---|---|---|---|---|---|
| Ranking list and panel definition according to PPLS-DA TOP 30 (200 runs) based on all antigens (NMO vs. MS). Data is shown only for antigens with freq >/= 100 or (freq >/= 80 and median rank </= 16). | | | | | |

| | ProteinID | GeneID | Gene.Name | freq | median rank |
|---|---|---|---|---|---|
| 4 | BBA00.068_1043138278 | 5982 | replication factor C (activator 1) 2,40kDa | 192 | 9 |
| 20 | BBA00.324_1043137810 | 27344 | proprotein convertase subtilisin/kexin type 1 inhibitor | 179 | 4 |
| 14 | BBA00.280_1043143162 | 11019 | lipoic acid synthetase | 170 | 13 |
| 5 | BBA00.080_1043143745 | No Gene ID | NA | 168 | 7 |
| 15 | BBA00.295_1043138939 | 1938 | eukaryotic translation elongation factor 2 | 166 | 10 |
| 2 | BBA00.035_1043138758 | 4775 | nuclear factor of activated T-cells, cytoplasmic, calcineurin-dependent 3 | 165 | 11 |
| 27 | BBA00.392_1043140856 | 23608 | makorin ring finger protein 1 | 163 | 7 |
| 1 | BBA00.027_1043138757 | 4155 | myelin basic protein | 157 | 12 |
| 11 | BBA00.242_0105509577 | 79155 | TNFAIP3 interacting protein 2 | 156 | 16 |
| 7 | BBA00.189_1043143648 | 84893 | F-box protein, helicase, 18 | 148 | 9 |
| 3 | BBA00.059_1043138765 | 58506 | SR-related CTD-associated factor 1 | 146 | 10 |
| 17 | BBA00.311_1043143356 | 5831 | pyrroline-5-carboxylate reductase 1 | 146 | 17 |
| 6 | BBA00.172_1043140859 | 119032 | chromosome 10 open reading frame 32 | 141 | 7 |
| 8 | BBA00.201_1043143257 | 2037 | erythrocyte membrane protein band 4.1-like 2 | 134 | 11 |
| 10 | BBA00.210_1043140481 | 64129 | tubulointerstitial nephritis antigen-like 1 | 129 | 8 |
| 29 | BBA00.400_1043140097 | 10081 | programmed cell death 7 | 125 | 16 |
| 18 | BBA00.317_1043144508 | 4122 | mannosidase, alpha, class 2A, member 2 | 123 | 20,5 |
| 28 | BBA00.395_1043138265 | 7416 | voltage-dependent anion channel 1 | 123 | 19 |
| 26 | BBA00.354_1043142586 | 7431 | vimentin | 119 | 17 |
| 19 | BBA00.323_1043135298 | 51510 | chromatin modifying protein 5 | 117 | 19 |
| 24 | BBA00.343_1043143350 | 6152 | ribosomal protein L24 | 117 | 17 |
| 16 | BBA00.302_1043135287 | 6130 | ribosomal protein L7a | 113 | 12 |
| 25 | BBA00.351_1043144220 | 128866 | chromatin modifying protein 4B | 113 | 17 |
| 23 | BBA00.335_1043143351 | 3068 | hepatoma-derived growth factor | 112 | 11 |
| 9 | BBA00.203_1043143552 | 4924 | nucleobindin 1 | 100 | 11 |
| 21 | BBA00.328_1043140473 | 6434 | transformer 2 beta homolog (Drosophila) | 94 | 13,5 |
| 12 | BBA00.252_1043144317 | 339230 | coiled-coil domain containing 137 | 93 | 15 |
| 13 | BBA00.263_1043138267 | 23646 | phospholipase D family, member 3 | 93 | 7 |
| 22 | BBA00.333_1043144502 | 375690 | WAS protein family homolog 5 pseudogene | 83 | 13,5 |

**Table 4.6 NMO vs MS**

| | | | | |
|---|---|---|---|---|
| Ranking list of PPLS-DA results based on antigens with freq >/= 100 or (freq >/= 80 and median rank </= 16). This ranking was obtained from a ranking list and panel definition according to PPLS-DA TOP 30 (200 runs) based on all antigens. | | | | |

| | ProteinID | GeneID | Gene.Name | abs.loading.weight.comp.1. |
|---|---|---|---|---|
| x.BBA00.260_1043136934 | BBA00.260_1043136934 | 361 | aquaporin 4 | 0,43483 |
| x.BBA00.324_1043137810 | BBA00.324_1043137810 | 27344 | proprotein convertase subtilisin/kexin type 1 inhibitor | 0,24482 |
| x.BBA00.392_1043140856 | BBA00.392_1043140856 | 23608 | makorin ring finger protein 1 | 0,22539 |
| x.BBA00.080_1043143745 | BBA00.080_1043143745 | No Gene ID | NA | 0,21189 |
| x.BBA00.189_1043143648 | BBA00.189_1043143648 | 84893 | F-box protein, helicase, 18 | 0,20617 |
| x.BBA00.035_1043138758 | BBA00.035_1043138758 | 4775 | nuclear factor of activated T-cells, cytoplasmic, calcineurin-dependent 3 | 0,19922 |
| x.BBA00.295_1043138939 | BBA00.295_1043138939 | 1938 | eukaryotic translation elongation factor 2 | 0,19893 |
| x.BBA00.059_1043138765 | BBA00.059_1043138765 | 58506 | SR-related CTD-associated factor 1 | 0,19819 |
| x.BBA00.068_1043138278 | BBA00.068_1043138278 | 5982 | replication factor C (activator 1) 2, 40kDa | 0,19374 |
| x.BBA00.027_1043138757 | BBA00.027_1043138757 | 4155 | myelin basic protein | 0,18682 |
| x.BBA00.335_1043143351 | BBA00.335_1043143351 | 3068 | hepatoma-derived growth factor | 0,17965 |
| x.BBA00.280_1043143162 | BBA00.280_1043143162 | 11019 | lipoic acid synthetase | 0,17895 |
| x.BBA00.311_1043143356 | BBA00.311_1043143356 | 5831 | pyrroline-5-carboxylate reductase 1 | 0,16419 |
| x.BBA00.400_1043140097 | BBA00.400_1043140097 | 10081 | programmed cell death 7 | 0,16210 |
| x.BBA00.354_1043142586 | BBA00.354_1043142586 | 7431 | vimentin | 0,16168 |
| x.BBA00.242_0105509577 | BBA00.242_0105509577 | 79155 | TNFAIP3 interacting protein 2 | 0,15911 |
| x.BBA00.333_1043144502 | BBA00.333_1043144502 | 375690 | WAS protein family homolog 5 pseudogene | 0,15897 |
| x.BBA00.351_1043144220 | BBA00.351_1043144220 | 128866 | chromatin modifying protein 4B | 0,15808 |
| x.BBA00.328_1043140473 | BBA00.328_1043140473 | 6434 | transformer 2 beta homolog (Drosophila) | 0,15124 |
| x.BBA00.343_1043143350 | BBA00.343_1043143350 | 6152 | ribosomal protein L24 | 0,14977 |
| x.BBA00.172_1043140859 | BBA00.172_1043140859 | 119032 | chromosome 10 open reading frame 32 | 0,14915 |
| x.BBA00.201_1043143257 | BBA00.201_1043143257 | 2037 | erythrocyte membrane protein band 4.1-like 2 | 0,14367 |
| x.BBA00.210_1043140481 | BBA00.210_1043140481 | 64129 | tubulointerstitial nephritis antigen-like 1 | 0,14364 |
| x.BBA00.323_1043135298 | BBA00.323_1043135298 | 51510 | chromatin modifying protein 5 | 0,14331 |
| x.BBA00.317_1043144508 | BBA00.317_1043144508 | 4122 | mannosidase, alpha, class 2A, member 2 | 0,13510 |
| x.BBA00.395_1043138265 | BBA00.395_1043138265 | 7416 | voltage-dependent anion channel 1 | 0,12820 |
| x.BBA00.302_1043135287 | BBA00.302_1043135287 | 6130 | ribosomal protein L7a | 0,12354 |
| x.BBA00.203_1043143552 | BBA00.203_1043143552 | 4924 | nucleobindin 1 | 0,11654 |
| x.BBA00.263_1043138267 | BBA00.263_1043138267 | 23646 | phospholipase D family, member 3 | 0,09830 |

**Table 4.7 NMO vs MS**

| | | | | | |
|---|---|---|---|---|---|
| Antigens (without AQP-4) with freq >/= 100 or (freq >/= 80 and median rank </= 16. This data was obtained from a ranking list and panel definition according to PPLS-DA TOP 30 (200 runs) based on all antigens without AQP-4. | | | | | |

| | ProteinID | GeneID | Gene.Name | freq | median rank |
|---|---|---|---|---|---|
| 4 | BBA00.068_1043138278 | 5982 | replication factor C (activator 1) 2,40kDa | 192 | 9 |
| 20 | BBA00.324_1043137810 | 27344 | proprotein convertase subtilisin/kexin type 1 inhibitor | 179 | 4 |
| 14 | BBA00.280_1043143162 | 11019 | lipoic acid synthetase | 170 | 13 |
| 5 | BBA00.080_1043143745 | No Gene ID | NA | 168 | 7 |
| 15 | BBA00.295_1043138939 | 1938 | eukaryotic translation elongation factor 2 | 166 | 10 |
| 2 | BBA00.035_1043138758 | 4775 | nuclear factor of activated T-cells, cytoplasmic, calcineurin-dependent 3 | 165 | 11 |
| 27 | BBA00.392_1043140856 | 23608 | makorin ring finger protein 1 | 163 | 7 |
| 1 | BBA00.027_1043138757 | 4155 | myelin basic protein | 157 | 12 |
| 11 | BBA00.242_0105509577 | 79155 | TNFAIP3 interacting protein 2 | 156 | 16 |
| 7 | BBA00.189_1043143648 | 84893 | F-box protein, helicase, 18 | 148 | 9 |
| 3 | BBA00.059_1043138765 | 58506 | SR-related CTD-associated factor 1 | 146 | 10 |
| 17 | BBA00.311_1043143356 | 5831 | pyrroline-5-carboxylate reductase 1 | 146 | 17 |
| 6 | BBA00.172_1043140859 | 119032 | chromosome 10 open reading frame 32 | 141 | 7 |
| 8 | BBA00.201_1043143257 | 2037 | erythrocyte membrane protein band 4.1-like 2 | 134 | 11 |
| 10 | BBA00.210_1043140481 | 64129 | tubulointerstitial nephritis antigen-like 1 | 129 | 8 |
| 29 | BBA00.400_1043140097 | 10081 | programmed cell death 7 | 125 | 16 |
| 18 | BBA00.317_1043144508 | 4122 | mannosidase, alpha, class 2A, member 2 | 123 | 20,5 |
| 28 | BBA00.395_1043138265 | 7416 | voltage-dependent anion channel 1 | 123 | 19 |
| 26 | BBA00.354_1043142586 | 7431 | vimentin | 119 | 17 |
| 19 | BBA00.323_1043135298 | 51510 | chromatin modifying protein 5 | 117 | 19 |
| 24 | BBA00.343_1043143350 | 6152 | ribosomal protein L24 | 117 | 17 |
| 16 | BBA00.302_1043135287 | 6130 | ribosomal protein L7a | 113 | 12 |
| 25 | BBA00.351_1043144220 | 128866 | chromatin modifying protein 4B | 113 | 17 |
| 23 | BBA00.335_1043143351 | 3068 | hepatoma-derived growth factor | 112 | 11 |
| 9 | BBA00.203_1043143552 | 4924 | nucleobindin 1 | 100 | 11 |
| 21 | BBA00.328_1043140473 | 6434 | transformer 2 beta homolog (Drosophila) | 94 | 13,5 |
| 12 | BBA00.252_1043144317 | 339230 | coiled-coil domain containing 137 | 93 | 15 |
| 13 | BBA00.263_1043138267 | 23646 | phospholipase D family, member 3 | 93 | 7 |
| 22 | BBA00.333_1043144502 | 375690 | WAS protein family homolog 5 pseudogene | 83 | 13,5 |

**Table 4.8 NMO vs MS**

| | | | | |
|---|---|---|---|---|
| Ranking list of PPLS-DA results based on antigens (without AQP-4) with freq >/= 100 or (freq >/= 80 and median rank </= 16). The data was obtained from ranking list and panel definition according to PPLS-DA TOP 30 (200 runs) based on all antigens without AQP-4. | | | | |

| | ProteinID | GeneID | Gene.Name | abs.loading.weight.comp.1. |
|---|---|---|---|---|
| x.BBA00.324_1043137810 | BBA00.324_1043137810 | 27344 | proprotein convertase subtilisin/kexin type 1 inhibitor | 0,27926 |
| x.BBA00.392_1043140856 | BBA00.392_1043140856 | 23608 | makorin ring finger protein 1 | 0,27348 |
| x.BBA00.189_1043143648 | BBA00.189_1043143648 | 84893 | F-box protein, helicase, 18 | 0,24841 |
| x.BBA00.080_1043143745 | BBA00.080_1043143745 | No Gene ID | NA | 0,23627 |
| x.BBA00.059_1043138765 | BBA00.059_1043138765 | 58506 | SR-related CTD-associated factor 1 | 0,23544 |
| x.BBA00.335_1043143351 | BBA00.335_1043143351 | 3068 | hepatoma-derived growth factor | 0,22899 |
| x.BBA00.295_1043138939 | BBA00.295_1043138939 | 1938 | eukaryotic translation elongation factor 2 | 0,22314 |
| x.BBA00.035_1043138758 | BBA00.035_1043138758 | 4775 | nuclear factor of activated T-cells, cytoplasmic, calcineurin-dependent 3 | 0,21771 |
| x.BBA00.027_1043138757 | BBA00.027_1043138757 | 4155 | myelin basic protein | 0,20552 |
| x.BBA00.068_1043138278 | BBA00.068_1043138278 | 5982 | replication factor C (activator 1) 2, 40kDa | 0,19991 |
| x.BBA00.333_1043144502 | BBA00.333_1043144502 | 375690 | WAS protein family homolog 5 pseudogene | 0,19416 |
| x.BBA00.280_1043143162 | BBA00.280_1043143162 | 11019 | lipoic acid synthetase | 0,19006 |
| x.BBA00.354_1043142586 | BBA00.354_1043142586 | 7431 | vimentin | 0,18441 |
| x.BBA00.328_1043140473 | BBA00.328_1043140473 | 6434 | transformer 2 beta homolog (Drosophila) | 0,17953 |
| x.BBA00.400_1043140097 | BBA00.400_1043140097 | 10081 | programmed cell death 7 | 0,17719 |
| x.BBA00.311_1043143356 | BBA00.311_1043143356 | 5831 | pyrroline-5-carboxylate reductase 1 | 0,17545 |
| x.BBA00.351_1043144220 | BBA00.351_1043144220 | 128866 | chromatin modifying protein 4B | 0,17456 |
| x.BBA00.242_0105509577 | BBA00.242_0105509577 | 79155 | TNFAIP3 interacting protein 2 | 0,16367 |
| x.BBA00.343_1043143350 | BBA00.343_1043143350 | 6152 | ribosomal protein L24 | 0,15860 |
| x.BBA00.323_1043135298 | BBA00.323_1043135298 | 51510 | chromatin modifying protein 5 | 0,15775 |
| x.BBA00.172_1043140859 | BBA00.172_1043140859 | 119032 | chromosome 10 open reading frame 32 | 0,13622 |
| x.BBA00.317_1043144508 | BBA00.317_1043144508 | 4122 | mannosidase, alpha, class 2A, member 2 | 0,13588 |
| x.BBA00.201_1043143257 | BBA00.201_1043143257 | 2037 | erythrocyte membrane protein band 4.1-like 2 | 0,13389 |
| x. B BA00.210 1043140481 | BBA00.210_1043140481 | 64129 | tubulointerstitial nephritis antigen-like 1 | 0,13054 |
| x.BBA00.395_1043138265 | BBA00.395_1043138265 | 7416 | voltage-dependent anion channel 1 | 0,12765 |
| x.BBA00.252_1043144317 | BBA00.252_1043144317 | 339230 | coiled-coil domain containing 137 | 0,11655 |
| x.BBA00.302_1043135287 | BBA00.302_1043135287 | 6130 | ribosomal protein L7a | 0,11465 |
| x.BBA00.203_1043143552 | BBA00.203_1043143552 | 4924 | nucleobindin 1 | 0,10603 |
| x.BBA00.263_1043138267 | BBA00.263_1043138267 | 23646 | phospholipase D family, member 3 | 0,08149 |

### Combined Analysis Results:

**Table 5.1 NMO vs healthy controls**

| | | | | |
|---|---|---|---|---|
| Overlap of panels from univariate (Ranking based on scoring and "edge candidates" resulting from volcano plot) and multivariate analysis (Ranking based on PPLS-DA) with AQP-4. | | | | |

| | ProteinID | GeneID | BBA Set | Gene Name |
|---|---|---|---|---|
| 1 | BBA00.047_1043136648 | 627 | BBA00 | brain-derived neurotrophic factor |
| 2 | BBA00.059_1043138765 | 58506 | BBA00 | SR-related CTD-associated factor 1 |
| 3 | BBA00.068_1043138278 | 5982 | BBA00 | replication factor C (activator 1) 2, 40kDa |
| 4 | BBA00.075_1043137831 | 5370 | BBA00 | pro-melanin-concentrating hormone-like 2, pseudogene |
| 5 | BBA00.109_1043137791 | 3915 | BBA00 | laminin, gamma 1 (formerly LAMB2) |
| 6 | BBA00.119_1043136825 | 324 | BBA00 | adenomatous polyposis coli |
| 7 | BBA00.140_1043143542 | 5819 | BBA00 | poliovirus receptor-related 2 (herpesvirus entry mediator B) |
| 8 | BBA00.205_1043143644 | 57455 | BBA00 | REX1, RNA exonuclease 1 homolog (S. cerevisiae) |
| 9 | BBA00.260_1043136934 | 361 | BBA00 | aquaporin 4 |
| 10 | BBA00.288_1043143161 | 10436 | BBA00 | EMG1 nucleolar protein homolog (S. cerevisiae) |
| 11 | BBA00.298_1043143832 | 55827 | BBA00 | DDB1 and CUL4 associated factor 6 |
| 12 | BBA00.311_1043143356 | 5831 | BBA00 | pyrroline-5-carboxylate reductase 1 |
| 13 | BBA00.315_1043143926 | 10445 | BBA00 | microspherule protein 1 |
| 14 | BBA00.347_1043135290 | 11054 | BBA00 | opioid growth factor receptor |
| 15 | BBA00.365_1043144408 | 3320 | BBA00 | heat shock protein 90kDa alpha (cytosolic), class A member 1 |
| 16 | BBA00.392_1043140856 | 23608 | BBA00 | makorin ring finger protein 1 |

**Table 5.2 NMO vs MS**

| | | | | |
|---|---|---|---|---|
| Overlap of panels from univariate (Ranking based on scoring and "edge candidates" resulting from volcano plot) and multivariate analysis (Ranking based on PPLS-DA) with AQP-4. | | | | |

| | ProteinID | GeneID | BBA Set | Gene Name |
|---|---|---|---|---|
| 1 | BBA00.027_1043138757 | 4155 | BBA00 | myelin basic protein |
| 2 | BBA00.035_1043138758 | 4775 | BBA00 | nuclear factor of activated T-cells, cytoplasmic, calcineurin-dependent 3 |
| 3 | BBA00.068_1043138278 | 5982 | BBA00 | replication factor C (activator 1) 2, 40kDa |
| 4 | BBA00.080_1043143745 | No Gene ID | BBA00 | NA |
| 5 | BBA00.210_1043140481 | 64129 | BBA00 | tubulointerstitial nephritis antigen-like 1 |
| 6 | BBA00.260_1043136934 | 361 | BBA00 | aquaporin 4 |
| 7 | BBA00.288_1043143161 | 10436 | BBA00 | EMG1 nucleolar protein homolog (S. cerevisiae) |
| 8 | BBA00.295_1043138939 | 1938 | BBA00 | eukaryotic translation elongation factor 2 |
| 9 | BBA00.324_1043137810 | 27344 | BBA00 | proprotein convertase subtilisin/kexin type 1 inhibitor |
| 10 | BBA00.328_1043140473 | 6434 | BBA00 | transformer 2 beta homolog (Drosophila) |
| 11 | BBA0O.340_104313893 | 65109 | BBA00 | UPF3 regulator of nonsense transcripts homolog B (yeast) |
| 12 | BBA00.343_1043143350 | 6152 | BBA00 | ribosomal protein L24 |
| 13 | BBA00.376_1043143543 | 6838 | BBA00 | surfeit 6 |
| 14 | BBA00.392_1043140856 | 23608 | BBA00 | makorin ring finger protein 1 |

**Table 5.3 NMO vs healthy controls**

| | | | | |
|---|---|---|---|---|
| Overlap of panels from univariate (Ranking based on scoring and "edge candidates" resulting from volcano plot) and multivariate analysis (Ranking based on PPLS-DA) without AQP-4. | | | | |

| | ProteinID | GeneID | BBA Set | Gene Name |
|---|---|---|---|---|
| 1 | BBA00.044_1043136937 | 25841 | BBA00 | ankyrin repeat and BTB (POZ) domain containing 2 |
| 2 | BBA00.047_1043136648 | 627 | BBA00 | brain-derived neurotrophic factor |
| 3 | BBA00.059_1043138765 | 58506 | BBA00 | SR-related CTD-associated factor 1 |
| 4 | BBA00.068_1043138278 | 5982 | BBA00 | replication factor C (activator 1) 2, 40kDa |
| 5 | BBA00.075_1043137831 | 5370 | BBA00 | pro-melanin-concentrating hormone-like 2, pseudogene |
| 6 | BBA00.109_1043137791 | 3915 | BBA00 | laminin, gamma 1 (formerly LAMB2) |
| 7 | BBA00.119_1043136825 | 324 | BBA00 | adenomatous polyposis coli |
| 8 | BBA00.140_1043143542 | 5819 | BBA00 | poliovirus receptor-related 2 (herpesvirus entry mediator B) |
| 9 | BBA00.205_1043143644 | 57455 | BBA00 | REX1, RNA exonuclease 1 homolog (S. cerevisiae) |
| 10 | BBA00.288_1043143161 | 10436 | BBA00 | EMG1 nucleolar protein homolog (S. cerevisiae) |
| 11 | BBA00.298_1043143832 | 55827 | BBA00 | DDB1 and CUL4 associated factor 6 |
| 12 | BBA00.311_1043143356 | 5831 | BBA00 | pyrroline-5-carboxylate reductase 1 |
| 13 | BBA00.315_1043143926 | 10445 | BBA00 | microspherule protein 1 |
| 14 | BBA00.347_1043135290 | 11054 | BBA00 | opioid growth factor receptor |
| 15 | BBA00.351_1043144220 | 128866 | BBA00 | chromatin modifying protein 4B |
| 16 | BBA00.365_1043144408 | 3320 | BBA00 | heat shock protein 90kDa alpha (cytosolic), class A member 1 |
| 17 | BBA00.392_1043140856 | 23608 | BBA00 | makorin ring finger protein 1 |

**Table 5.4 NMO vs MS**

| | | | | |
|---|---|---|---|---|
| Overlap of panels from univariate (Ranking based on scoring and "edge candidates" resulting from volcano plot) and multivariate analysis (Ranking based on PPLS-DA) without AQP-4. | | | | |

| | ProteinID | GeneID | BBA Set | Gene Name |
|---|---|---|---|---|
| 1 | BBA00.027_1043138757 | 4155 | BBA00 | myelin basic protein |
| 2 | BBA00.035_1043138758 | 4775 | BBA00 | nuclear factor of activated T-cells, cytoplasmic, calcineurin-dependent 3 |
| 3 | BBA00.068_1043138278 | 5982 | BBA00 | replication factor C (activator 1) 2, 40kDa |
| 4 | BBA00.080_1043143745 | No Gene ID | BBA00 | NA |
| 5 | BBA00.210_1043140481 | 64129 | BBA00 | tubulointerstitial nephritis antigen-like 1 |
| 6 | BBA00.288_1043143161 | 10436 | BBA00 | EMG1 nucleolar protein homolog (S. cerevisiae) |
| 7 | BBA00.295_1043138939 | 1938 | BBA00 | eukaryotic translation elongation factor 2 |
| 8 | BBA00.324_1043137810 | 27344 | BBA00 | proprotein convertase subtilisin/kexin type 1 inhibitor |
| 9 | BBA00.328_1043140473 | 6434 | BBA00 | transformer 2 beta homolog (Drosophila) |
| 10 | BBA00.340_1043138937 | 65109 | BBA00 | UPF3 regulator of nonsense transcripts homolog B (yeast) |
| 11 | BBA00.343_1043143350 | 6152 | BBA00 | ribosomal protein L24 |
| 12 | BBA00.376_1043143543 | 6838 | BBA00 | surfeit 6 |
| 13 | BBA00.392_1043140856 | 23608 | BBA00 | makorin ring finger protein 1 |

**Table 5.5 NMO vs healthy controls**

| | | | | |
|---|---|---|---|---|
| Union of panels from univariate and multivariate analysis. | | | | |

| | ProteinID | GeneID | BBA Set | Gene Name |
|---|---|---|---|---|
| 1 | BBA00.032_1043143271 | 57136 | BBA00 | chromosome 20 open reading frame 3 |
| 2 | BBA00.036_1043143937 | 5864 | BBA00 | RAB3A, member RAS oncogene family |
| 3 | BBA00.044_1043136937 | 25841 | BBA00 | ankyrin repeat and BTB (POZ) domain containing 2 |
| 4 | BBA00.047_1043136648 | 627 | BBA00 | brain-derived neurotrophic factor |
| 5 | BBA00.059_1043138765 | 58506 | BBA00 | SR-related CTD-associated factor 1 |
| 6 | BBA00.065_1043140868 | 79582 | BBA00 | sperm associated antigen 16 |
| 7 | BBA00.066_1043142796 | 10075 | BBA00 | HECT, UBA and WWE domain containing 1 |
| 8 | BBA00.068_1043138278 | 5982 | BBA00 | replication factor C (activator 1) 2, 40kDa |
| 9 | BBA00.075_1043137831 | 5370 | BBA00 | pro-melanin-concentrating hormone-like 2, pseudogene |
| 10 | BBA00.080_1043143745 | No Gene ID | BBA00 | NA |
| 11 | BBA00.109_1043137791 | 3915 | BBA00 | laminin, gamma 1 (formerly LAMB2) |
| 12 | BBA00.119_1043136825 | 324 | BBA00 | adenomatous polyposis coli |
| 13 | BBA00.134_1043141344 | 4744 | BBA00 | neurofilament, heavy polypeptide |
| 14 | BBA00.140_1043143542 | 5819 | BBA00 | poliovirus receptor-related 2 (herpesvirus entry mediator B) |
| 15 | BBA00.150_1043141334 | 7204 | BBA00 | triple functional domain (PTPRF interacting) |
| 16 | BBA00.198_1043136831 | 2935 | BBA00 | G1 to S phase transition 1 |
| 17 | BBA00.201_1043143257 | 2037 | BBA00 | erythrocyte membrane protein band 4.1-like 2 |
| 18 | BBA00.205_1043143644 | 57455 | BBA00 | REX1, RNA exonuclease 1 homolog (S. cerevisiae) |
| 19 | BBA00.210_1043140481 | 64129 | BBA00 | tubulointerstitial nephritis antigen-like 1 |
| 20 | BBA00.231_1043135293 | 10539 | BBA00 | glutaredoxin 3 |
| 21 | BBA00.237_1043135291 | 5595 | BBA00 | mitogen-activated protein kinase 3 |
| 22 | BBA00.251_1043141343 | 80728 | BBA00 | Rho GTPase activating protein 39 |
| 23 | BBA00.260_1043136934 | 361 | BBA00 | aquaporin 4 |
| 24 | BBA00.272_1043143163 | No Gene ID | BBA00 | NA |
| 25 | BBA00.284_1043136634 | 1270 | BBA00 | ciliary neurotrophic factor |
| 26 | BBA00.286_1043143930 | 4354 | BBA00 | membrane protein, palmitoylated 1, 55kDa |
| 27 | BBA00.288_1043143161 | 10436 | BBA00 | EMG1 nucleolar protein homolog (S. cerevisiae) |
| 28 | BBA00.298_1043143832 | 55827 | BBA00 | DDB1 and CUL4 associated factor 6 |
| 29 | BBA00.308_1043136631 | 23114 | BBA00 | neurofascin |
| 30 | BBA00.311_1043143356 | 5831 | BBA00 | pyrroline-5-carboxylate reductase 1 |
| 31 | BBA00.315_1043143926 | 10445 | BBA00 | microspherule protein 1 |
| 32 | BBA00.320_1043140474 | 8874 | BBA00 | Rho guanine nucleotide exchange factor (GEF) 7 |
| 33 | BBA00.340_1043138937 | 65109 | BBA00 | UPF3 regulator of nonsense transcripts homolog B (yeast) |
| 34 | BBA00.341_1043144416 | 7316 | BBA00 | ubiquitin C |
| 35 | BBA00.347_1043135290 | 11054 | BBA00 | opioid growth factor receptor |
| 36 | BBA00.351_1043144220 | 128866 | BBA00 | chromatin modifying protein 4B |
| 37 | BBA00.365_1043144408 | 3320 | BBA00 | heat shock protein 90kDa alpha (cytosolic), class A member 1 |
| 38 | BBA00.392_1043140856 | 23608 | BBA00 | makorin ring finger protein 1 |

**Table 5.6 NMO vs MS**

| | | | | |
|---|---|---|---|---|
| Union of panels from univariate and multivariate analysis. | | | | |

| | ProteinID | GeneID | BBA Set | Gene Name |
|---|---|---|---|---|
| 1 | BBA00.027_1043138757 | 4155 | BBA00 | myelin basic protein |
| 2 | BBA00.035_1043138758 | 4775 | BBA00 | nuclear factor of activated T-cells, cytoplasmic, calcineurin-dependent 3 |
| 3 | BBA00.059_1043138765 | 58506 | BBA00 | SR-related CTD-associated factor 1 |
| 4 | BBA00.068_1043138278 | 5982 | BBA00 | replication factor C (activator 1) 2, 40kDa |
| 5 | BBA00.080_1043143745 | No Gene ID | BBA00 | NA |
| 6 | BBA00.119_1043136825 | 324 | BBA00 | adenomatous polyposis coli |
| 7 | BBA00.169_1043144025 | 90861 | BBA00 | hematological and neurological expressed 1-like |
| 8 | BBAOO.172-1043140859 | 119032 | BBA00 | chromosome 10 open reading frame 32 |
| 9 | BBA00.189_1043143648 | 84893 | BBA00 | F-box protein, helicase, 18 |
| 10 | BBA00.193_1043143261 | 10569 | BBA00 | SLU7 splicing factor homolog (S. cerevisiae) |
| 11 | BBA00.201_1043143257 | 2037 | BBA00 | erythrocyte membrane protein band 4.1-like 2 |
| 12 | BBA00.203_1043143552 | 4924 | BBA00 | nucleobindin 1 |
| 13 | BBA00.205_1043143644 | 57455 | BBA00 | REX1, RNA exonuclease 1 homolog (S. cerevisiae) |
| 14 | BBA00.210_1043140481 | 64129 | BBA00 | tubulointerstitial nephritis antigen-like 1 |
| 15 | BBA00.213_1043140091 | 6461 | BBA00 | Src homology 2 domain containing adaptor protein B |
| 16 | BBA00.242_0105509577 | 79155 | BBA00 | TNFAIP3 interacting protein 2 |
| 17 | BBA00.252_1043144317 | 339230 | BBA00 | coiled-coil domain containing 137 |
| 18 | BBA00.260_1043136934 | 361 | BBA00 | aquaporin 4 |
| 19 | BBA00.263_1043138267 | 23646 | BBA00 | phospholipase D family, member 3 |
| 20 | BBA00.280_1043143162 | 11019 | BBA00 | lipoic acid synthetase |
| 21 | BBA00.288_1043143161 | 10436 | BBA00 | EMG1 nucleolar protein homolog (S. cerevisiae) |
| 22 | BBA00.295_1043138939 | 1938 | BBA00 | eukaryotic translation elongation factor 2 |
| 23 | BBA00.302_1043135287 | 6130 | BBA00 | ribosomal protein L7a |
| 24 | BBA00.311_1043143356 | 5831 | BBA00 | pyrroline-5-carboxylate reductase 1 |
| 25 | BBA00.317_1043144508 | 4122 | BBA00 | mannosidase, alpha, class 2A, member 2 |
| 26 | BBA00.323_1043135298 | 51510 | BBA00 | chromatin modifying protein 5 |
| 27 | BBA00.324_1043137810 | 27344 | BBA00 | proprotein convertase subtilisin/kexin type 1 inhibitor |
| 28 | BBA00.328_1043140473 | 6434 | BBA00 | transformer 2 beta homolog (Drosophila) |
| 29 | BBA00.333_1043144502 | 375690 | BBA00 | WAS protein family homolog 5 pseudogene |
| 30 | BBA00.335_1043143351 | 3068 | BBA00 | hepatoma-derived growth factor |
| 31 | BBA00.340_1043138937 | 65109 | BBA00 | UPF3 regulator of nonsense transcripts homolog B (yeast) |
| 32 | BBA00.343_1043143350 | 6152 | BBA00 | ribosomal protein L24 |
| 33 | BBA00.351_1043144220 | 128866 | BBA00 | chromatin modifying protein 4B |
| 34 | BBA00.354_1043142586 | 7431 | BBA00 | vimentin |
| 35 | BBA00.376_1043143543 | 6838 | BBA00 | surfeit 6 |
| 36 | BBA00.392_1043140856 | 23608 | BBA00 | makorin ring finger protein 1 |
| 37 | BBA00.395_1043138265 | 7416 | BBA00 | voltage-dependent anion channel 1 |
| 38 | BBA00.400_1043140097 | 10081 | BBA00 | programmed cell death 7 |

### References

(1) Kuhle J, Petzold A (2011). What makes a prognostic biomarker in CNS diseases: strategies for targeted biomarker discovery? Part 2: chronic progressive and relapsing disease. Expert Opinion on Medical Diagnostics, Volume 5, Number 5, September, pp. 393-410(18).
(2) Lennon VA, Wingerchuk DM, Kryzer TJ, Pittock SJ, Lucchinetti CF, Fujihara K, Nakashima I, Weinshenker BG (2004). A serum autoantibody marker of neuromyelitis optica: distinction from multiple sclerosis. Lancet 364:2106-2112.
(3) Wingerchuk DM, Lennon VA, Pittock SJ, Lucchinetti CF, Weinshenker BG. Revised diagnostic criteria for neuromyelitis optica. Neurology. 2006 May 23; 66(10):1485-9.
(4) Fazio R , Malosio ML, Lampasona V, De Feo D, Privitera D, Marnetto F, Centonze D, Ghezzi A, Comi G, Furlan R and Martino G (2009). Antiacquaporin 4 antibodies detection by different techniques in neuromyelitis optica patients. Multiple Sclerosis 15(10), pp. 1153-1163.
(5) Waters P, Vincent A (2008). Detection of anti-aquaporin-4 antibodies in neuromyelitis optica: current status of the assays. Int MS J. 2008 Sep;15(3):99-105.
(6) Benjamini Y, Hochberg Y (1995). Controlling the false discovery rate: a practical and powerful approach to multiple testing. Journal of the Royal Statistical Society B, Vol. 57, 289-300.

## Claims

1. Method for identifying markers for Neuromelitis Optica (NMO) comprising the steps
a) Expose a marker candidate for NMO to sample(s) of NMO patient(s), measure the bonding of the marker candidate by immunofluorescent assay and determine the median fluorescence intensity (MFI) for the marker candidate;
b) Expose the same marker candidate to control sample(s), measure the bonding of the marker candidate by immunofluorescent assay and determine the median fluorescence intensity (MFI) for the marker candidate;
c) Process MFI data from steps a) and b) by univariate analysis;
d) Process MFI data from steps a) and b) by multivariate analysis;
e) Combine the data obtained by univariate analysis and multivariate analysis and identify thereby marker(s) for NMO.

2. Method according to claim 1 wherein procession of MFI data is performed by univariate analysis based on EST (exploratory statistics and testing) and / or volcano plot.

3. Method according to claim 1 or 2 wherein procession of MFI data by multivariate analysis is performed by partial least squares discriminant analysis (PLS-DA) and/or powered PLS-DA.

4. Method according to claim 1 to 3 wherein univariate analysis of MFI data of a marker candidate comprises one or more parameters selected from p-value, fold change, effect size, Fisher's ratio, area under the curve (AUC), median absolute MFI within the group, the univariate Mann-Whitney U test.

5. Method according to claim 1 to 4 wherein control samples are selected from healthy persons.

6. Marker for NMO identified by a method according to claim 1 to 5 and selected from the group comprising SEQ ID No. 1 to 87, SEQ ID No. 88 to 175, SEQ ID No. 176 to 263, partial sequences of SEQ ID No. 1 to 263 and homologous of SEQ ID No. 1 to 263.

7. Marker for discriminating MNO from Multiple Sclerosis wherein the marker is identified by a method according to claim 1 to 5 and selected from the group comprising SEQ ID No. 1 to 44, SEQ ID No. 88 to 132, SEQ ID NO. 176 to 220, partial sequences and homologous thereof, preferably selected from the group of SEQ ID No. 1 to 16, SEQ ID No. 88 to 104, SEQ ID No. 176 to 192, partial sequences and homologous thereof.

8. Marker for discriminating NMO from the healthy state wherein the marker is identified by a method according to claim 1 to 5 and selected from the group comprising SEQ ID No. 45 to 87, SEQ ID No. 133 to 175, SEQ ID NO. 221 to 263, partial sequences and homologous thereof, preferably selected from the group of SEQ ID No. 45 - 63, SEQ ID No. 133 to 161, SEQ ID No. 221 to 239, partial sequences and homologous thereof.

9. Use of one or more marker(s) for NMO selected from the group comprising sequence SEQ ID No. 1 to 87, SEQ ID No. 88 to 175, SEQ ID No. 176 to 263, partial sequences of SEQ ID No. 1 to 263 and homologous of SEQ ID No. 1 to 263 as diagnostic agent, for use in diagnosis of MNO, for prognosis in NMO, for determination of treatment of NMO, for surveillance of treatment of MNO, for stratification in NMO, for therapy control or prediction of prognosis of NMO covering decisions for the treatment and therapy of the patient, in particular the hospitalization of a patient with NMO, for decision of use, effect and/or dosage of one or more drugs, for use as a therapeutic measure or the monitoring of the course of the disease and/or the course of therapy, for etiology or classification of NMO optionally together with prognosis, optionally together with one or more markers for NMO like for example AQP-4.

10. Diagnostic agent or test kit comprising one or more marker(s) for NMO selected from the group comprising sequence SEQ ID No. 1 to 87, SEQ ID No. 88 to 175, SEQ ID No. 176 to 263, partial sequences of SEQ ID No. 1 to 263 and homologous of SEQ ID No. 1 to 263 and optionally further substances and/or additives.

11. Panel of markers comprising one or more marker(s) for NMO selected from the group comprising sequence SEQ ID No. 1 to 87, SEQ ID No. 88 to 175, SEQ ID No. 176 to 263, partial sequences of SEQ ID No. 1 to 263 and homologous of SEQ ID No. 1 to 263.

12. Assay or protein array comprising a panel of marker(s) according to claim 11, **characterized in that** the marker(s) is/are applied to a solid support, in particular a filter, a membrane, a bead or microsphere like for example a magnetic or fluorophore-labeled bead, a silica wafer, glass, metal, ceramics, plastics, a chip, a target for mass spectrometry or a matrix.

13. Use of a panel of markers according claims 11 or an assay or protein array according to claim 12 for the identification and/or validation of an active agent for the prevention or treatment of NMO wherein the panel or the assay or protein array contains means for detecting a binding success, **characterized in that** the panel or assay or protein array a.) is brought into contact with at least one substance to be tested and b.) a binding success is detected.

14. Method for detecting MNO comprising the steps
a. providing at least one marker for NMO selected from the group comprising sequence SEQ ID No. 1 to 87, SEQ ID No. 88 to 175, SEQ ID No. 176 to 263, partial sequences of SEQ ID No. 1 to 263 and homologous of SEQ ID No. 1 to 263,
b. bringing the one or more marker(s) into contact with body fluid or tissue extract of a person, for example a patient and
c. detecting an interaction of the body fluid or tissue extract with the marker(s) from a.).

15. Target for the treatment and/or therapy of NMO selected from the group comprising sequence SEQ ID No. 1 to 87, SEQ ID No. 88 to 175, SEQ ID No. 176 to 263, partial sequences of SEQ ID No. 1 to 263 and homologous of SEQ ID No. 1 to 263.
